# FASCICULE DE BREVET EUROPEEN

(11) **EP 2 083 002 B1**
(45) Date de publication et mention de la délivrance du brevet: **23.03.2011**
(21) Numéro de dépôt: 08291199.1
(22) Date de dépôt: 16.12.2008
(51) Int. Cl.: C07C 2/10, C10G 50/00, B01J 21/12

(54) **Procédé d'oligomérisation des oléfines utilisant un catalyseur à base de silice-alumine**
Oligomerisierungsverfahren von Olefinen mit Hilfe eines Katalysators auf Silizium-Aluminium-Basis
Method for oligomerising olefins using a catalyst based on silica-alumina

(30) Priorité: 28.01.2008 FR 0800437
(43) Date de publication de la demande: 29.07.2009
(73) Titulaire: IFP Energies nouvelles, 92852 Rueil-Malmaison Cedex (FR)
(72) Inventeur: Coupard, Vincent, 69120 Vaulx en Velin (FR); Chaumonnot, Alexandra, 69008 Lyon (FR); Simon, Laurent, 69100 Villeurbanne (FR)

(56) Documents cités:
- FR-A- 2 873 116
- US-A1- 2005 177 017

## Description

### Domaine de l'invention

L'invention se rapporte à tout procédé d'oligomérisation des oléfines permettant la production de carburant, par exemple la production d'essence et/ou de kérosène, à partir de charges oléfiniques légères contenant entre 2 et 8 atomes de carbone, et en particulier à partir de charges oléfiniques légères contenant une forte proportion de propylène et/ou de butènes et/ou de pentènes utilisant un catalyseur d'oligomérisation à base de silice-alumine à teneur réduite en macropores.

### Art Antérieur

Les procédés d'oligomérisation des oléfines légères destinés à produire des oléfines de plus haut poids moléculaire sont largement utilisés en raffinage et pétrochimie dans le but de valoriser les oléfines légères en bases pour carburants de type essence, kérosène ou gazole, ou bien en solvants. Ces réactions d'oligomérisation sont conduites en présence d'un catalyseur, le plus souvent un catalyseur solide. Les oléfines se combinent en dimères, trimères, tétramères, etc., le degré d'oligomérisation dépendant du type de catalyseur utilisé et des conditions opératoires de température, de pression et de débit de charge. L'avantage du procédé d'oligomérisation, par rapport à d'autres procédés bien connus dans le domaine du raffinage et de la pétrochimie conduisant à la même gamme de produits, réside dans le fait que les produits obtenus ne contiennent pas de soufre et contiennent très peu de composés aromatiques. Les catalyseurs d'oligomérisation solides souvent cités dans la littérature sont des catalyseurs de type acide phosphorique solide (US 2.913.506 et US 3.661.801), silice-alumine (US 4.197.185, US 4.544.791 et EP 0.463.673), zéolithe (US 4.642.404 et US 5.284.989) ou bien, dans une moindre mesure, de type hétéropolyanion (IN 170903).

Les catalyseurs de type acide phosphorique solide présentent une bonne activité en oligomérisation mais sont difficiles à manipuler, en particulier au moment du déchargement de l'unité associée au procédé, car ils ont tendance à prendre en masse en présence d'oléfines. De plus, ils ne sont pas régénérables. Les catalyseurs de type hétéropolyanion conduisent à un degré de polymérisation limité car ils supportent mal des températures élevées. Les zéolithes conduisent à des oligomères à taux de branchement plus limité que les catalyseurs précédents de par leur sélectivité de forme dans les micropores. Ceci est favorable pour la production de gazole devant présenter un indice de cétane correct mais peu favorable par exemple à la production d'essence devant présenter un bon indice d'octane. Enfin, les catalyseurs de type silice-alumine cités dans la littérature présentent des caractéristiques de porosité assez variables qui engendrent des réactivités différentes. Par exemple le brevet EP 0.463.673 enseigne, pour l'oligomérisation du propylène, l'utilisation d'une silice-alumine amorphe présentant un rapport molaire SiO₂/Al₂O₃ compris entre 30 et 500, une surface spécifique entre 500 et 1000 m²/g, un volume poreux total entre 0,3 et 0,6 ml/g, un diamètre de pore moyen au plus égal à environ 1 nm, et aucun pore présentant un diamètre supérieur à 3 nm. Le brevet US 4.544.791 enseigne, pour l'oligomérisation d'oléfines en C4, l'utilisation d'une silice-alumine amorphe avec une teneur massique en silice entre 60 et 95%, une surface spécifique entre 50 et 500 m²/g, un volume poreux total entre 0,4 et 0,9 ml/g, ladite silice-alumine ne présentant pas de phase d'alumine en diffraction des rayons X.

La demande de brevet US 2006/0063955 divulgue un catalyseur dont le support est de type silice-alumine non zéolithique pour l'oligomérisation des oléfines légères contenant entre 5 et 95% poids de silice et qui a une surface spécifique comprise entre 100 et 550 m²/g et un volume poreux mesuré par porosimétrie au mercure et par porosimétrie à l'azote compris entre 0,1 et 0,6 ml/g.

### Résumé de l'invention

La demande de brevet FR-A-2.873.116 décrit un procédé d'oligomérisation des oléfines légères ayant de 2 à 8 atomes de carbone pour la production de carburant en présence d'un catalyseur comprenant un support non-zéolithique à base de silice-alumine contenant une quantité supérieure à 5% poids et inférieure ou égale à 95% poids de silice. Ledit catalyseur est préparé en procédant d'abord au mélange d'un composé d'alumine partiellement soluble en milieu acide avec un composé de silice totalement soluble ou avec une combinaison totalement soluble d'alumine et de silice hydratées puis à un traitement hydrothermal, par exemple un vapotraitement, ou un traitement thermique.

La présente invention a pour objet un procédé d'oligomérisation d'une charge hydrocarbonée oléfinique consistant en la mise en contact de ladite charge avec un catalyseur comprenant une silice - alumine, la teneur massique en silice dudit catalyseur étant comprise entre 5 et 95% poids, ledit catalyseur étant préparé selon un procédé comprenant au moins :
a) le mélange d'au moins un composé aluminique partiellement soluble en milieu acide avec soit au moins un composé silicique totalement soluble dans le mélange réactionnel soit une combinaison formée d'au moins un composé silicique et d'au moins un composé aluminique, lesdits composés silicique et aluminique étant totalement solubles dans le mélange réactionnel, de manière à former un solide précurseur dudit catalyseur
b) le traitement hydrothermal du solide issu de ladite étape a) par calcination sous air humide pendant une durée comprise entre 4 et 7 heures.

### Intérêt de l'invention

La mise en présence d'au moins un composé aluminique partiellement soluble en milieu acide avec soit au moins un composé silicique totalement soluble dans le mélange réactionnel soit une combinaison formée d'au moins un composé silicique et d'au moins un composé aluminique lesquels sont entièrement solubles dans le mélange réactionnel constitue une première étape visant à développer des interactions spécifiques entre les espèces siliciques et aluminiques. Le traitement hydrothermal réalisé en présence d'eau en phase liquide ou en phase vapeur, pendant une durée d'au moins 3 heures, consécutif au mélange visé à l'étape a) du procédé de préparation du catalyseur constitue une deuxième étape permettant de contrôler le degré d'intimité entre les espèces aluminiques et siliciques et donc d'assurer une homogénéité du catalyseur à base de silice-alumine élaboré selon le procédé de préparation. Ceci se traduit par des propriétés d'acidité et des propriétés texturales particulières qui conduisent à de meilleurs performances catalytiques notamment en terme de sélectivité lors de son utilisation comme catalyseur dans un procédé d'oligomérisation des oléfines.

### Description de l'invention

Dans la suite du texte on entend par oligomérisation toute réaction d'addition d'une oléfine sur une autre oléfine.

La présente invention a pour objet un procédé d'oligomérisation d'une charge hydrocarbonée oléfinique consistant en la mise en contact de ladite charge avec un catalyseur comprenant une silice - alumine, la teneur massique en silice dudit catalyseur étant comprise entre 5 et 95% poids, ledit catalyseur étant préparé selon un procédé comprenant au moins :
a) le mélange d'au moins un composé aluminique partiellement soluble en milieu acide avec soit au moins un composé silicique totalement soluble dans le mélange réactionnel soit une combinaison formée d'au moins un composé silicique et d'au moins un composé aluminique, lesdits composés silicique et aluminique étant totalement solubles dans le mélange réactionnel, de manière à former un solide précurseur dudit catalyseur
b) le traitement hydrothermal du solide issu de ladite étape a) par calcination sous air humide pendant une durée comprise entre 4 et 7 heures.

Le catalyseur utilisé dans le procédé d'oligomérisation selon la présente invention est un catalyseur non-zéolitique à base d'une silice-alumine, c'est-à-dire comprenant de la silice et de l'alumine. Les caractéristiques de la silice-alumine présente dans le catalyseur d'oligomérisation sont les suivantes :
- la teneur massique en silice (SiO₂) est comprise entre 5% et 95% poids, de préférence comprise entre 10 et 80% poids, de manière plus préférée comprise entre 20% et 80% poids et de manière encore plus préférée comprise entre 25% et 75% poids.
- la teneur en impuretés cationiques est généralement inférieure à 0,1% poids, de manière préférée inférieure à 0,05% poids et de manière encore plus préférée inférieure à 0,025% poids. On entend par teneur en impuretés cationiques la teneur totale en alcalins, en particulier en sodium.
- la teneur en impuretés anioniques est généralement inférieure à 1% poids, de manière préférée inférieure à 0,5% poids et de manière encore plus préférée inférieure à 0,1% poids. Les impuretés anioniques présentes dans ledit catalyseur d'oligomérisation sont notamment des halogénures, en particulier des chlorures, ainsi que des sulfates et des nitrates.
- le diamètre moyen poreux, noté D_{moyen}, du catalyseur, mesuré par intrusion au porosimètre à mercure, est compris entre 20 et 140 Å, de préférence entre 40 et 120 Å et de manière encore plus préférée entre 50 et 100 Å,
   -- le rapport entre le volume V2, mesuré par intrusion au porosimètre à mercure et occupé par les pores de diamètre compris entre le D_{moyen} - 30 Å et le D_{moyen} +30 Å, sur le volume poreux total, également mesuré par intrusion au porosimètre à mercure, est supérieur à 0,6, de préférence supérieur à 0,7 et de manière encore plus préférée supérieur à 0,8,
- le volume V3 occupé par les pores de diamètre supérieur à D_{moyen} +30 Å, mesuré par intrusion au porosimètre à mercure, est inférieur à 0,1 ml/g, de manière préférée inférieur à 0,06 ml/g et de manière encore plus préférée inférieur à 0,04 ml/g,
- le volume poreux total, mesuré par intrusion au porosimètre à mercure, est compris entre 0,6 ml/g et 0,9 ml/g, de manière préférée compris entre 0,65 et 0,9 ml/g et de manière encore plus préférée entre 0,7 et 0,9 ml/g,
- le volume poreux total, mesuré par isotherme d'adsorption à l'azote, est compris entre 0,6 ml/g et 0,9 ml/g, de manière préférée compris entre 0,65 et 0,9 ml/g et de manière encore plus préférée entre 0,7 et 0,9 ml/g,
- la surface spécifique BET est comprise entre 100 et 550 m²/g, de préférence comprise entre 150 et 500 m²/g, de manière plus préférée comprise entre 150 et 350 m²/g et de manière encore plus préférée comprise entre 150 et 250 m²/g,
   -- la surface d'adsorption, laquelle est définie à partir de la branche de l'hystérésis à l'adsorption de l'azote de l'isotherme pour des pores de diamètre compris entre 3 et 200 nm, est telle que le rapport entre la surface d'adsorption et la surface BET soit supérieur à 0,5, de manière préférée supérieur à 0,65 et de manière plus préférée supérieur à 0,8,
- le volume poreux, mesuré par intrusion au porosimètre à mercure, compris dans les pores de diamètre supérieur à 160 Å est inférieur à 0,06 ml/g, de préférence inférieur à 0,05 ml/g et de manière encore plus préférée inférieur à 0,01 ml/g,
- le volume poreux, mesuré par intrusion au porosimètre à mercure, compris dans les pores de diamètre supérieur à 200 Å est inférieur à 0,06 ml/g, de préférence inférieur à 0,05 ml/g et de manière encore plus préférée inférieur à 0,01 ml/g,
- le volume poreux, mesuré par intrusion au porosimètre à mercure, compris dans les pores de diamètre supérieur à 500 Å est inférieur à 0,06 ml/g, de préférence inférieur à 0,05 ml/g, de manière plus préférée inférieur à 0,02 ml/g et de manière encore plus préférée inférieur à 0,01 ml/g,
- le diagramme de diffraction X du catalyseur d'oligomérisation contient au moins les raies principales caractéristiques d'au moins une des alumines de transition comprises dans le groupe composé par les alumines alpha, rhô, khi, kappa, êta, gamma, thêta et delta et de manière préférée au moins les raies principales caractéristiques d'au moins une des alumines de transition comprise dans le groupe composé par l'alumine gamma, êta, thêta et delta, et de manière plus préférée au moins les raies principales caractéristiques de l'alumine gamma ou êta, et de manière encore plus préférée le diagramme contient les pics à un d compris entre 1,39 et 1,40 Å et à un d compris entre 1,97 Å et 2,00 Å.

La densité de remplissage tassée du catalyseur d'oligomérisation est supérieure à 0,40 g/cm³, de manière préférée supérieure à 0,45 g/cm³, de manière très préférée supérieure à 0,50 g/cm³.

La silice-alumine présente dans le catalyseur utilisé dans le procédé selon l'invention est de préférence une silice-alumine homogène à l'échelle du micromètre et dans laquelle la teneur en impuretés cationiques, notamment en cation Na⁺, est inférieure à 0,1% poids, de manière préférée inférieure à 0,05% poids et de manière encore plus préférée inférieure à 0,025% poids et la teneur en impuretés anioniques, par exemple en SO₄²⁻ ou en Cl⁻, est inférieure à 1% poids, de manière préférée inférieure à 0,5% poids et de manière encore plus préférée inférieure à 0,1% poids.

Ainsi tout procédé de synthèse de silice-alumine connu de l'Homme du métier conduisant à une silice-alumine possédant les propriétés d'acidité et les propriétés texturales définies ci-dessus, caractérisée en particulier par une homogénéité des espèces siliciques et aluminiques à l'échelle du micromètre, voire à l'échelle du nanomètre, et pour laquelle les impuretés cationiques (par exemple Na⁺) peuvent être ramenées à moins de 0,1% poids, de manière préférée à une teneur inférieure à 0,05% poids et de manière encore plus préférée inférieure à 0,025% poids et pour laquelle les impuretés anioniques, par exemple les anions SO₄²⁻ et Cl⁻, peuvent être ramenées à moins de 1% poids, de manière préférée à une teneur inférieure à 0,5% poids et de manière encore plus préférée à une teneur inférieure à 0,1 % poids, convient pour préparer le catalyseur d'oligomérisation utilisé dans le procédé de l'invention.

Dans un mode de réalisation du catalyseur d'oligomérisation utilisé dans le procédé de l'invention, ledit catalyseur contient au moins deux zones silico-aluminiques, lesdites zones ayant des rapports molaires Si/Al inférieurs ou supérieurs au rapport molaire Si/Al global déterminé par fluorescence X. Ainsi un catalyseur ayant un rapport molaire Si/Al global égal à 0,5 comprend par exemple deux zones silico-aluminiques, l'une des zones ayant un rapport molaire Si/Al déterminé par MET inférieur à 0,5 et l'autre zone ayant un rapport molaire Si/Al déterminé par MET compris entre 0,5 et 2,5.

Dans un autre mode de réalisation du catalyseur d'oligomérisation utilisé dans le procédé de l'invention, le catalyseur contient une seule zone silico-aluminique, ladite zone ayant un rapport molaire Si/Al égal au rapport molaire Si/Al global déterminé par fluorescence X et inférieur à 6.

Conformément à l'invention et selon un mode de réalisation préférée du catalyseur d'oligomérisation utilisé dans le procédé de l'invention, ledit catalyseur est constitué intégralement de ladite silice-alumine ; il est dépourvu de tout autre élément.

Les spectres RMN MAS du solide de ²⁷Al du catalyseur d'oligomérisation à base de silice-alumine utilisé dans le procédé de invention montrent deux massifs de pics distincts. Un premier type d'aluminium dont le maximum résonne vers 10 ppm s'étend entre -100 et 20 ppm. La position du maximum suggère que ces espèces sont essentiellement de type Al_{VI} (octaédrique). Un deuxième type d'aluminium minoritaire dont le maximum résonne vers 60 ppm s'étend entre 20 et 110 ppm. Ce massif peut être décomposé en au moins deux espèces. L'espèce prédominante de ce massif correspondrait aux atomes d'Al_{IV} (tétraédrique). Pour le catalyseur d'oligomérisation à base de silice-alumine utilisé dans le procédé de la présente invention, la proportion des Al_{VI} octaédriques est avantageusement supérieure à 50% molaire, de manière préférée supérieure à 60% molaire et de manière encore plus préférée supérieure à 70% molaire.

L'acidité du catalyseur d'oligomérisation utilisé dans le procédé selon l'invention est avantageusement mesurée par suivi IR de la thermodésorption de la pyridine. Généralement, le rapport B/L, tel que décrit ci-après dans la présente description, du catalyseur d'oligomérisation utilisé dans le procédé selon l'invention est compris entre 0,05 et 6 et de manière préférée entre 0,5 et 2.

Le catalyseur d'oligomérisation utilisé dans le procédé de l'invention peut éventuellement contenir au moins un élément métallique choisi parmi les métaux des groupes IVB, VB, VIB et VIII. Parmi les métaux du groupe IVB, le titane, le zirconium et/ou l'hafnium peuvent être présents dans le catalyseur. Parmi les métaux du groupe VB, le vanadium, le niobium et/ou le tantale peuvent être présents dans le catalyseur. Parmi les métaux du groupe VIB, le chrome, le molybdène et/ou le tungstène peuvent être présents dans le catalyseur. Parmi les métaux du groupe VIII, les métaux appartenant à la première ligne des métaux du groupe VIII, à savoir le fer, le cobalt et le nickel, sont préférés. La teneur de ces métaux peut aller jusqu'à 10% poids du catalyseur final. Le catalyseur peut éventuellement contenir également du silicium comme élément dopant déposé sur la silice-alumine.

Le demandeur a découvert que le catalyseur à base de silice-alumine obtenu à partir d'un mélange d'au moins un composé aluminique partiellement soluble en milieu acide avec soit au moins un composé silicique totalement soluble dans le mélange réactionnel soit une combinaison formée d'au moins un composé silicique et d'au moins un composé aluminique, lesdits composés silicique et aluminique étant totalement solubles dans le mélange réactionnel, suivi d'une homogénéisation à l'échelle micrométrique, voire à l'échelle nanométrique dudit mélange via un traitement hydrothermal d'une durée d'au moins 3 heures, permet d'obtenir un catalyseur particulièrement sélectif pour la mise en oeuvre du procédé d'oligomérisation selon l'invention. En effet, la mise en présence d'au moins un composé aluminique partiellement soluble en milieu acide avec soit au moins un composé silicique totalement soluble dans le mélange réactionnel soit une combinaison formée d'au moins un composé silicique et d'au moins un composé aluminique lesquels sont entièrement solubles dans le mélange réactionnel correspond à la mise en présence dans ledit mélange visé à l'étape a) du procédé de préparation du catalyseur d'espèces aluminiques et siliciques de taille et de réactivité chimique spécifiques conduisant de ce fait à des interactions contrôlées entre ces espèces à l'origine, pour partie, de l'homogénéité de la silice-alumine présente dans le catalyseur utilisé pour la mise en oeuvre du procédé d'oligomérisation selon l'invention. En fonction de la nature chimique des composés aluminiques et siliciques utilisés pour la préparation du catalyseur, le contrôle du degré d'interactivité entre les espèces siliciques et aluminiques peut être réalisé à toutes les étapes du procédé de préparation précédent l'étape de traitement hydrothermal. Pour exemple et de façon non exhaustive, le mélange d'un composé aluminique partiellement soluble du type hydrate d'aluminium Al₂O₃, nH₂O (boehmite) avec un composé silicique totalement soluble de type acide orthosilicique décationisé peut se faire en milieu aqueux sous l'influence de divers paramètres opératoires de synthèse contrôlés (pH, température, etc.) ou bien le mélange d'un composé aluminique partiellement soluble du type hydrate d'aluminium Al₂O₃, nH₂O (boehmite) avec un composé silicique totalement soluble de type solution colloïdale de silice commercial (Ludox®) peut se faire lors de l'étape de mise en forme consécutivement au travail mécanique généré lors de ce procédé de mise en forme. Pour finir, le traitement hydrothermal réalisé en présence d'eau - en phase vapeur ou en phase liquide - consécutif au mélange visé à l'étape a) du procédé de préparation du catalyseur d'oligomérisation permet finalement d'assurer le degré d'homogénéité à l'échelle micrométrique voire nanométrique entre les espèces aluminiques et siliciques nécessaire au développement des propriétés d'acidité et des propriétés texturales du catalyseur utilisé dans le procédé d'oligomérisation des oléfines selon l'invention.

Conformément à un premier mode de réalisation de l'étape a) du procédé de préparation du catalyseur d'oligomérisation utilisé dans le procédé de l'invention, on mélange au moins un composé aluminique partiellement soluble en milieu acide avec au moins un composé silicique totalement soluble dans le mélange réactionnel. Les sources du composé silicique totalement soluble mis en présence avec au moins un composé aluminique partiellement soluble en milieu acide sont avantageusement choisies dans le groupe formé par l'acide silicique, les solutions colloïdales d'acide silicique, les silicates alcalins hydrosolubles et les sels cationiques de silicium, par exemple le métasilicate de sodium hydraté, le Ludox® sous forme ammoniacale ou sous forme alcaline et les silicates d'ammonium quaternaire. Les solutions colloïdales d'acide silicique peuvent être préparées selon l'une des méthodes connues de l'Homme du métier. De manière préférée, on utilise comme source de composé silicique totalement soluble une solution d'acide orthosilicique décationisé laquelle est préparée à partir d'un silicate alcalin hydrosoluble par échange ionique sur une résine. Conformément à un deuxième mode de réalisation de l'étape a) du procédé de préparation du catalyseur d'oligomérisation utilisé dans le procédé de l'invention, on mélange au moins un composé aluminique partiellement soluble en milieu acide avec une combinaison formée d'au moins un composé silicique et d'au moins un composé aluminique, lesdits composés silicique et aluminique étant totalement solubles dans le mélange réactionnel. Parmi les sources de ladite combinaison, les silice-alumines hydratées totalement solubles sont avantageusement utilisées. Elles sont préférentiellement préparées par coprécipitation vraie en conditions opératoires stationnaires maîtrisées (pH, concentration, température, temps de séjour moyen) ou par réaction d'une solution basique contenant le silicium, par exemple sous forme de silicate de sodium, optionnellement de l'aluminium, par exemple sous forme d'aluminate de sodium, avec une solution acide contenant au moins un sel d'aluminium, par exemple le sulfate d'aluminium. Au moins un carbonate ou encore du CO₂ peut éventuellement être rajouté au milieu réactionnel quel que soit le mode de préparation.

Par coprécipitation vraie, on entend un procédé par lequel au moins un composé aluminique et au moins un composé silicique totalement solubles en milieu basique ou acide comme décrits ci-après, sont mis en contact, simultanément ou séquentiellement, en présence d'au moins un composé précipitant et/ou coprécipitant de façon à obtenir une phase mixte essentiellement constituée de silice-alumine hydratée, laquelle est éventuellement homogénéisée par agitation intense, cisaillement, broyage colloïdal ou encore par combinaison de ces opérations unitaires. Par exemple, ces silice-alumines hydratées peuvent avoir été préparées selon les enseignements des brevets américains US 2.908.635, US 3.423.332, US 3.433.747, US 3.451.947, US 3.629.152 et US 3.650.988.

Conformément à l'étape a) du procédé de préparation du catalyseur d'oligomérisation utilisé dans le procédé d'oligomérisation de l'invention, au moins un composé silicique totalement soluble dans le mélange réactionnel ou une combinaison formée d'au moins un composé silicique et d'au moins un composé aluminique, lesdits composés silicique et aluminique étant totalement solubles dans le mélange réactionnel, est mélangé(e) avec au moins un composé aluminique partiellement soluble en milieu acide. La propriété de dissolution totale dans le mélange réactionnel dudit composé silicique ou desdits composés silicique et aluminique formant ladite combinaison a été évaluée de manière approchée selon la méthode suivante. Une quantité fixée (15 g) du composé silicique ou de ladite combinaison, préférentiellement hydratée, est introduite dans un milieu aqueux de pH préétabli. De manière préférée, la concentration de solide, à savoir de composé silicique ou de composés silicique et aluminique, rapporté par litre de suspension est de 0,2 mole par litre. Le pH de la solution est au moins de 12 et il peut être obtenu par utilisation d'une source alcaline. De manière préférée, il est intéressant d'utiliser NaOH. Le mélange est ensuite agité mécaniquement par un agitateur à turbine de type défloculeuse pendant 30 minutes à 800 t/min. Une fois l'agitation terminée, le mélange est centrifugé 10 minutes à 3000 t/min. Le gâteau est séparé du liquide surnageant : la solution est filtrée sur un filtre de porosité 4 de diamètre 19 cm. Le séchage et la calcination à 1000°C des 2 fractions, à savoir celle du gâteau et celle du liquide surnageant, sont ensuite réalisés. Le rapport R obtenu en divisant la masse du solide équivalente au gâteau par la masse de solide présent dans le liquide surnageant est alors défini. Par totalement soluble, on entend un rapport R au moins supérieur à 0,9.

Pour la mise en oeuvre de l'étape a) du procédé de préparation du catalyseur d'oligomérisation utilisé dans le procédé de l'invention, on utilise un composé aluminique partiellement soluble en milieu acide avantageusement choisi dans le groupe des composés aluminiques de formule générale Al₂O₃, nH₂O (n ≤ 5) et ayant une surface spécifique comprise entre 150 et 600 m²/g. Il est possible en particulier d'utiliser des composés hydratés d'alumine tels que : l'hydrargillite, la gibbsite, la bayerite, la boehmite, la pseudo-boehmite et les gels d'alumine amorphe ou essentiellement amorphe. Il est également envisageable de mettre en oeuvre les formes déshydratées de ces composés qui sont constitués d'alumines de transition et qui comportent au moins une des phases prises dans le groupe suivant : alpha, rhô, khi, êta, gamma, kappa, thêta, et delta, qui se différencient les unes des autres essentiellement par l'organisation de leur structure cristalline.

Par "partiellement soluble en milieu acide", il est entendu que la mise en contact dudit composé aluminique avec une solution acide, par exemple d'acide nitrique ou d'acide sulfurique, provoque sa dissolution partielle avant toute addition soit d'au moins un composé silicique totalement soluble dans le mélange réactionnel soit d'une combinaison formé d'au moins un composé silicique et d'au moins un composé aluminique, lesdits composés silicique et aluminique étant totalement solubles dans le mélange réactionnel.

Cette propriété de dissolution partielle est une propriété recherchée pour la préparation du catalyseur mis en oeuvre dans le procédé de l'invention. Elle s'applique aux poudres d'alumine hydratées, aux poudres atomisées d'alumine hydratées, aux dispersions ou suspensions d'alumine hydratées ou à l'une quelconque de leur combinaison, avant une quelconque addition d'un composé contenant tout ou en partie du silicium.

Ladite propriété de dissolution partielle dudit composé aluminique a été évaluée de manière approchée selon la méthode suivante. Une quantité précise du composé aluminique en poudre ou en suspension est introduite dans un milieu aqueux de pH préétabli. Le mélange est ensuite agité mécaniquement. Une fois l'agitation terminée, le mélange est laissé sans agitation durant 24 heures. De manière préférée, la concentration de solide en Al₂O₃ rapportée par litre de suspension est de 0,5 mole par litre. Le pH de la solution de la suspension est de 2 et est obtenu soit par utilisation de HNO₃, soit par utilisation de HCl, soit par utilisation de HClO₄. De manière préférée, il est intéressant d'utiliser HNO₃. La répartition de l'aluminium est telle qu'une première partie de l'aluminium est présente dans une fraction sédimentée et qu'une deuxième partie de l'aluminium est présente dans une fraction dissoute. La répartition de l'aluminium dans chacune de ces deux fractions a été suivie par dosage de l'aluminium par absorption UV. La fraction dissoute encore appelée surnageant a été ultrafiltrée (membrane de polyéther-sulfones, Millipore NMWL 30000) et digérée dans de l'acide concentré. La quantité d'aluminium dans ladite fraction dissoute (surnageant) correspond au composé aluminique non-sédimenté et à l'aluminium dissous et la fraction ultrafiltrée correspond à l'aluminium dissous uniquement. La quantité de particules sédimentées est déduite de la concentration théorique en aluminium dans la suspension (en considérant que tout le solide introduit est en suspension) et des quantités d'aluminium non-sédimentées en suspension et d'aluminium dissous en solution. La présence de particules sédimentées caractérise la propriété de dissolution partielle.

Les précurseurs aluminiques utilisés pour leur propriété de dissolution partielle dans la préparation du catalyseur mis en oeuvre dans le procédé de la présente invention se distinguent donc de ceux utilisés dans le cas des coprécipitations vraies, qui sont entièrement solubles en milieu acide, comme les sels cationiques d'alumine dont le nitrate d'aluminium par exemple, ou en milieu basique. Les méthodes utilisant lesdits précurseurs aluminiques utilisés pour leur propriété de dissolution partielle se distinguent des coprécipitations vraies car l'un des éléments, en l'espèce le composé aluminique, est partiellement soluble.

L'hydrate d'aluminium Al₂O₃, nH₂O utilisé de manière plus préférentielle est la boehmite, la pseudo-boehmite et les gels d'alumine amorphe ou essentiellement amorphe. Un mélange de ces produits sous quelque combinaison que ce soit peut être également utilisé. La boehmite est généralement décrite comme un monohydrate d'aluminium de formule Al₂O₃, nH₂O qui englobe en réalité un large spectre de matériaux de degrés d'hydratation et d'organisation variables avec des frontières plus ou moins bien définies : la boehmite gélatineuse la plus hydratée, avec n pouvant être supérieur à 2, la pseudo-boehmite ou la boehmite micro-cristalline avec n compris entre 1 et 2, puis la boehmite cristalline et enfin la boehmite bien cristallisée en gros cristaux avec n voisin de 1. La morphologie du monohydrate d'aluminium peut varier dans de larges limites entre ces deux formes extrêmes, aciculaire et prismatique. Tout un ensemble de formes variables peut être utilisé entre ces deux formes : chaîne, bateaux, plaquettes entrelacées. La préparation et/ou la mise en forme de solide à base d'alumine de transition issues de monohydrate d'aluminium est décrite en particulier dans les brevets US 3.520.654, US 3.630.670, US 3.864.461, US 4.154.812, US 4.313.923, DE 3.243.193 et US 4.371.513.

Des hydrates d'aluminium relativement purs peuvent être utilisés sous forme de poudre amorphe ou de poudre cristallisée ou de poudre cristallisée contenant une partie amorphe. L'hydrate d'aluminium peut également être introduit sous forme de suspensions ou dispersions aqueuses. Les suspensions ou dispersions aqueuses d'hydrate d'aluminium mise en oeuvre pour la préparation du catalyseur utilisé dans le procédé de l'invention peuvent être gélifiables ou coagulables. Les dispersions ou suspensions aqueuses peuvent également être obtenues ainsi qu'il est bien connu de l'Homme du métier par peptisation dans l'eau ou dans une solution aqueuse acide d'hydrates d'aluminium.

La dispersion d'hydrate d'aluminium peut être réalisée par tout procédé connu de l'Homme du métier : dans un réacteur en "batch", un mélangeur en continu, un malaxeur, un broyeur colloïdal. Un tel mélange peut être également réalisé dans un réacteur à écoulement piston et, notamment dans un mélangeur statique. On peut citer comme exemple les réacteurs Lightnin.

En outre, il est également possible d'utiliser comme source de composé aluminique partiellement soluble en milieu acide une alumine ayant été soumise au préalable à un traitement susceptible d'améliorer son degré de dispersion. A titre d'exemple, la dispersion de la source aluminique pourra être améliorée par un traitement d'homogénéisation mécanique préliminaire.

Les dispersions ou suspensions aqueuses d'alumine que l'on peut mettre en oeuvre sont notamment les suspensions ou dispersions aqueuses de boehmites fines ou ultra-fines qui sont composées de particules ayant des dimensions dans le domaine colloïdal. Les boehmites fines ou ultra-fines mises en oeuvre dans le cadre de la présente invention peuvent notamment avoir été obtenues selon les brevets FR 1.261.182 et FR 1.381.282 ou selon la demande de brevet européen EP 151.96. Il est également possible de mettre en oeuvre les suspensions ou dispersions aqueuses obtenues à partir de pseudo-boehmite, de gels d'alumine amorphe, de gels d'hydroxyde d'aluminium ou d'hydrargillite ultra-fine.

Le monohydrate d'aluminium peut être acheté parmi une variété de sources commerciales d'alumine telle que notamment les PURAL®, CATAPAL®, DISPERAL®, DISPAL® commercialisée par la société SASOL ou encore HIQ® commercialisée par ALCOA, ou bien être obtenu selon les méthodes de synthèse connues de l'Homme du métier : par déshydratation partielle de trihydrate d'aluminium par des méthodes conventionnelles ou par précipitation. Lorsque les alumines se présentent sous forme d'un gel, elles sont peptisées par l'eau ou par une solution aqueuse acide. Pour la technique par précipitation, la source acide peut être par exemple au moins un des composés suivants : le chlorure d'aluminium, le sulfate d'aluminium et le nitrate d'aluminium. La source basique d'aluminium peut être choisie parmi les sels basiques d'aluminium tels que l'aluminate de sodium et l'aluminate de potassium. Comme agents précipitants, l'hydroxyde de sodium, le carbonate de sodium, la potasse et l'ammoniaque peuvent être utilisés. Les agents précipitants sont choisis de telle manière que la source aluminique selon la présente invention et ces agents soient précipités ensemble. Selon la nature acide ou basique du composé de départ à base d'aluminium, l'hydrate d'aluminium est précipité à l'aide d'une base ou d'un acide choisi par exemple parmi l'acide chlorhydrique, l'acide sulfurique, la soude ou un composé basique ou acide de l'aluminium tel que cités ci-dessus. Les deux réactifs peuvent être le sulfate d'aluminium et l'aluminate de soude. Pour un exemple de préparation de alpha-monohydrate d'aluminium utilisant le sulfate d'aluminium et l'aluminate de soude, il est possible notamment de se référer au brevet US 4.154.812. La pseudo-boehmite peut notamment avoir été préparée selon le procédé décrit dans le brevet US 3.630.670 par réaction d'une solution d'aluminate alcalin avec une solution d'un acide minéral. Elle peut également avoir été préparée comme décrit dans le brevet FR 1.357.830. Les gels d'alumine amorphe peuvent notamment avoir été préparés selon les procédés décrits dans l'article "Alcoa Paper", 1972, 19, 9 et notamment par réaction d'un aluminate ou d'un sel d'aluminium ou par hydrolyse d'alcoolates d'aluminium ou par hydrolyse de sels basiques d'aluminium. Les gels d'hydroxyde d'aluminium peuvent notamment être ceux qui ont été préparés selon les procédés décrits dans les brevets américains US 3.268.295 et US 3.245.919 ou dans la demande de brevet WO 00/01617, par mélange d'une source acide d'aluminium et d'une base ou d'une source basique d'aluminium et d'un acide de manière à précipiter un monohydrate d'alumine, ledit mélange étant réalisé sans rétromélange. L'hydrargilite ultra-fine peut notamment avoir été préparée selon le procédé décrit dans le brevet US 1.371.808, par évolution à une température comprise entre la température ambiante et 60°C de gels d'alumine sous forme de gâteau.

Il est également possible d'utiliser comme sources de composé aluminique partiellement soluble en milieu acide les suspensions ou dispersions aqueuses de boehmite ou de pseudo-boehmite ultra-pures préparées selon un procédé dans lequel on effectue la réaction d'un aluminate alcalin avec de l'anhydride carbonique pour former un précipité d'hydroxycarbonate d'aluminium amorphe. Le précipité est obtenu par filtration puis lavé. Un tel procédé est notamment décrit dans le brevet US 3.268.295. Ensuite, 1) dans une première étape, le précipité lavé d'hydroxycarbonate d'aluminium amorphe est mélangé avec une solution acide, une base ou un sel ou de leurs mélanges (ce mélange est effectué en versant la solution sur l'hydroxycarbonate, le pH du milieu ainsi constitué étant inférieur à 11), 2) dans une deuxième étape, le milieu réactionnel ainsi constitué est chauffé à une température inférieure à 90°C pendant un temps d'au moins 5 minutes et 3) dans une troisième étape, le milieu résultant de la deuxième étape est chauffé à une température comprise entre 90°C et 250°C. Les dispersions ou suspensions de boehmite et pseudo-boehmite obtenus selon ce procédé présentent une teneur en alcalins inférieure à 0,005% exprimée sous forme de rapport pondéral oxyde du métal alcalin /Al₂O₃.

Pour un catalyseur à base de silice-alumine très pur, des suspensions ou dispersions de boehmites ou de pseudo-boehmites ultra-pures qui ont été obtenues selon le procédé décrit ci-dessus, ou des gels d'hydroxyde d'aluminium qui ont été préparés à partir de l'hydrolyse des alcoolates d'aluminium selon un procédé du type décrit dans le brevet US 2.892.858 sont utilisés de préférence.

On décrit sommairement le procédé de fabrication qui conduit à de tels gels d'hydroxyde d'aluminium de type boehmite obtenue comme sous-produit dans la fabrication de l'alcool par hydrolyse d'un alcoolate ou alcoxyde d'aluminium (synthèse de Ziegler). Les réactions de synthèse d'alcools Ziegler sont décrites notamment dans le brevet US 2.892.858. Selon ce procédé, le triéthylaluminium à partir d'aluminium, d'hydrogène et d'éthylène est tout d'abord préparé, la réaction étant réalisée en deux étapes avec recyclage partiel du triéthylaluminium. On ajoute de l'éthylène dans l'étape de polymérisation et on oxyde ensuite le produit obtenu en alcoolate d'aluminium, les alcools étant obtenus par hydrolyse. Les gels d'hydroxyde d'aluminium peuvent également être ceux qui ont été préparés selon les procédés décrits dans les brevets américains US 4.676.928 et US 6.030.599. L'alumine hydratée obtenue comme sous-produit de la réaction de Ziegler est notamment décrite dans un bulletin de la société CONOCO du 19 janvier 1971.

La dimension des particules d'alumine sous forme de poudre constituant la source d'alumine peut varier dans de larges limites. Elle est généralement comprise entre 1 et 100 microns.

### Méthodes de préparation du catalyseur d'oligomérisation

Le catalyseur utilisé dans le procédé d'oligomérisation selon l'invention est préparé selon un procédé de synthèse comportant au moins les deux étapes suivantes :
a) le mélange d'au moins un composé aluminique partiellement soluble en milieu acide avec soit au moins un composé silicique totalement soluble dans le mélange réactionnel soit une combinaison formée d'au moins un composé silicique et d'au moins un composé aluminique, lesdits composés silicique et aluminique présents dans ladite combinaison étant totalement solubles dans le mélange réactionnel, de manière à former un solide précurseur dudit catalyseur
b) le traitement hydrothermal, pendant une durée d'au moins 3 heures, du solide issu de ladite étape a).

Le mélange mentionné à l'étape a) du procédé de préparation du catalyseur utilisé dans le procédé d'oligomérisation selon l'invention peut être réalisé par exemple par l'une des méthodes décrites ci-après.

Un exemple de méthode de préparation dudit mélange visé à l'étape a) du procédé de préparation du catalyseur utilisé dans le procédé d'oligomérisation de l'invention consiste à préparer, à partir d'un silicate alcalin hydrosoluble, une solution d'acide orthosilicique (H₂SiO₄, H₂O) décationisée par échange ionique puis à l'ajouter à un mélange constitué d'un sel cationique d'aluminium en solution (par exemple le nitrate) et d'ammoniaque dans des conditions opératoires contrôlées ou à l'ajouter dans un premier temps au sel cationique d'aluminium en solution et à coprécipiter ensuite la solution ainsi obtenue par de l'ammoniaque dans des conditions opératoires contrôlées, les deux options possibles conduisant à un produit homogène, à savoir un hydrogel de silice-alumine. Cet hydrogel de silice-alumine, combinaison d'un composé aluminique et d'un composé silicique parfaitement soluble, est mélangé avec de la poudre ou une suspension d'hydrate d'aluminium, composé aluminique partiellement soluble en milieu acide. Après filtration, lavage et séchage suivi d'une mise en forme éventuelle, un solide précurseur du catalyseur d'oligomérisation est obtenu. Il est prêt pour subir le traitement hydrothermal visé à l'étape b) du procédé de préparation du catalyseur d'oligomérisation.

Un autre exemple de méthode de préparation dudit mélange visé à l'étape a) du procédé de préparation du catalyseur utilisé dans le procédé d'oligomérisation de l'invention consiste à précipiter l'hydrate d'alumine tel que décrit ci-dessus, à le filtrer et le laver, puis à le mélanger avec l'acide orthosilicique aqueux qui est employé comme composé silicique totalement soluble de façon à obtenir une suspension, laquelle est intimement homogénéisée mécaniquement par forte agitation et cisaillement. Une turbine Ultraturrax® ou encore une turbine Staro® peut être utilisée, ou encore un broyeur colloïdal, par exemple un broyeur colloïdal Staro®. La suspension homogène est alors séchée par atomisation, puis éventuellement calcinée entre 500 et 1200°C pendant au moins 3 heures et éventuellement mis en forme. Un solide précurseur du catalyseur est obtenu et est prêt pour subir le traitement hydrothermal en présence de vapeur d'eau visé à l'étape b) du procédé de préparation du catalyseur d'oligomérisation.

Un autre exemple de méthode permettant l'élaboration dudit mélange visé à l'étape a) du procédé de préparation du catalyseur utilisé dans le procédé d'oligomérisation de l'invention consiste à préparer comme ci-avant une solution décationisée d'acide orthosilicique (composé silicique totalement soluble) puis à l'ajouter simultanément ou consécutivement à un composé d'alumine partiellement soluble en milieu acide, par exemple un hydrate d'aluminium en poudre ou en suspension aqueuse acide. Afin d'augmenter le diamètre des pores du catalyseur silice-alumine final, au moins un composé basique peut éventuellement être rajouté au milieu réactionnel. Après une homogénéisation mécanique poussée de la suspension par agitation, ajustement éventuel par filtration de la teneur en matière sèche puis éventuellement ré-homogénéisation, le produit est séché avec possible mise en forme simultanément ou consécutivement, puis éventuellement calciné préférentiellement sous air, en four rotatif, à une température élevée et pendant un temps généralement d'au moins 2 heures avant de subir l'étape b) du procédé de préparation du catalyseur d'oligomérisation. Un autre exemple de méthode de préparation dudit mélange visé à l'étape a) du procédé de préparation du catalyseur utilisé dans le procédé d'oligomérisation de l'invention consiste à préparer une suspension ou une dispersion aqueuse d'alumine, par exemple un monohydrate d'aluminium (composé aluminique partiellement soluble en milieu acide), puis à l'ajouter simultanément ou consécutivement à un composé de silice totalement soluble, par exemple un silicate de sodium. Afin d'augmenter le diamètre des pores du catalyseur silice-alumine final, au moins un composé basique peut éventuellement être rajouté au milieu réactionnel. On procède ensuite à une filtration et au moins un lavage, éventuellement à au moins un lavage par une solution ammoniacale pour extraire par échange ionique le sodium résiduel. Après séchage avec éventuelle mise en forme puis éventuelle calcination comme ci-avant, un solide précurseur du catalyseur est obtenu. Il est prêt pour être soumis au traitement hydrothermal visé à l'étape b) du procédé de préparation du catalyseur d'oligomérisation.

Pour augmenter le diamètre des mésopores du catalyseur silice-alumine final, il peut être particulièrement avantageux comme l'enseigne le brevet US 4.066.574 de préparer ledit mélange visé à l'étape a) du procédé de préparation du catalyseur d'oligomérisation en réalisant une suspension ou une dispersion aqueuse d'alumine, par exemple un monohydrate d'aluminium (composé aluminique partiellement soluble en milieu acide), puis de la neutraliser par une solution basique, par exemple de l'ammoniaque, et enfin de l'ajouter simultanément ou consécutivement à un composé de silice totalement soluble, par exemple une solution décationisée d'acide orthosilicique. Après une homogénéisation mécanique poussée de la suspension par agitation intense, ajustement éventuel par filtration de la teneur en matière sèche puis ré-homogénéisation, le produit est séché avec possible mise en forme simultanément ou consécutivement et éventuellement calciné comme ci-avant. Un solide précurseur du catalyseur est ainsi obtenu. Il est prêt pour être soumis au traitement hydrothermal visé à l'étape b) du procédé de préparation du catalyseur d'oligomérisation.

Dans l'exposé des méthodes précitées, une première "homogénéisation" dudit mélange est souvent réalisé via des traitements mécaniques comme par exemple lors de la remise en solution d'un produit contenant une fraction solide comme une suspension, une poudre, un précipité filtré, suivi de sa dispersion sous agitation intense. L'homogénéisation mécanique d'une dispersion est un procédé bien connu de l'Homme du métier. Ladite homogénéisation peut être réalisée par tout procédé mécanique connu de l'Homme du métier, par exemple dans un réacteur en batch, un mélangeur en continu ou un malaxeur. Un tel mélange peut être réalisé dans un réacteur à écoulement piston et notamment dans un réacteur statique. On peut citer les réacteurs Lightnin. Une turbine Ultraturrax® ou encore une turbine Staro® peut être utilisée, ou encore un broyeur colloïdal par exemple, un broyeur colloïdal Staro®. Les broyeurs colloïdaux commerciaux IKA® peuvent être aussi utilisés.

Dans l'ensemble des méthodes précitées, il peut être éventuellement souhaitable d'ajouter, lors d'une étape quelconque de la préparation, une proportion mineure d'au moins un élément stabilisant choisi dans le groupe formé par la zircone et le titane. L'élément stabilisant est de préférence ajouté sous forme d'un sel soluble.

Conformément à l'étape b) du procédé de préparation du catalyseur utilisé dans le procédé d'oligomérisation selon l'invention, le traitement hydrothermal, réalisé pendant une durée comprise entre 4 et 7 heures, du solide issu de ladite étape a) décrite ci-dessus permet d'assurer l'homogénéité du support solide résultant de l'étape a) du procédé de préparation du catalyseur d'oligomérisation. Ledit traitement hydrothermal est effectué par calcination sous air humide. Ledit traitement hydrothermal consiste en la mise en contact à n'importe quelle étape de l'élaboration du solide issu de ladite étape a) décrite ci-dessus avec de l'eau, en phase vapeur ou en phase liquide. Sans que cela réduise la portée de l'invention, un tel traitement a pour effet de rendre mobile la composante silicique.

Ledit traitement hydrothermal par calcination sous air humide est réalisé dans un four en présence de vapeur d'eau. La température appliquée durant la calcination sous air humide est avantageusement comprise entre 600 et 1100°C et de préférence comprise entre 650°C et 800°C. La durée dudit traitement hydrothermal est comprise entre 4 et 7 heures. Ladite calcination sous air humide est réalisée en présence d'une teneur en vapeur d'eau supérieure à 20 g d'eau par kg d'air sec, de préférence supérieure à 40 g d'eau par kg d'air sec, de manière préférée supérieure à 100 g d'eau par kg d'air sec et de manière plus préférée supérieure à 300 g d'eau par kg d'air sec.

### Mise en forme du catalyseur

Le catalyseur d'oligomérisation utilisé dans le procédé selon l'invention se présente sous la forme de sphères, de pastilles ou d'extrudés, préférentiellement d'extrudés. De manière très avantageuse, ledit catalyseur d'oligomérisation se présente sous la forme d'extrudés de diamètre compris entre 0,5 et 5 mm et plus particulièrement entre 0,7 et 2,5 mm. Les formes sont cylindriques (qui peuvent être creuses ou non), cylindriques torsadés, multilobées (2, 3, 4 ou 5 lobes par exemple) ou anneaux. Les formes cylindriques et multilobées sont utilisées de manière préférée, mais toute autre forme peut être utilisée.

Le catalyseur d'oligomérisation utilisé dans le procédé de l'invention est obtenu par mise en forme de la silice-alumine par toute technique connue de l'Homme du métier. La mise en forme peut être réalisée par exemple par extrusion, par dragéification, par pastillage, par séchage, par atomisation, par la méthode de la coagulation en goutte (oil-drop), par granulation au plateau tournant ou par toute autre méthode bien connue de l'Homme du métier.

La mise en forme peut également être réalisée en présence des différents constituants du catalyseur et extrusion de la pâte minérale obtenue, par pastillage, mise en forme sous forme de billes au drageoir tournant ou au tambour, coagulation en goutte (oil-drop), ou tout autre procédé connu d'agglomération d'une poudre contenant de l'alumine et de la silice et éventuellement d'autres ingrédients choisis parmi ceux mentionnés ci-dessus.

Plus précisément, quand le catalyseur se présente sous la forme d'extrudés, on peut ajouter ou retirer de l'eau pour ajuster la viscosité de la pâte à extruder. Cette étape peut être réalisée à tout stade de l'étape de malaxage. Pour ajuster la teneur en matière solide de la pâte à extruder afin de la rendre extrudable, on peut également ajouter un composé majoritairement solide et de préférence un oxyde ou un hydrate. On utilisera de manière préférée un hydrate et de manière encore plus préférée un hydrate d'aluminium. La perte au feu de cet hydrate est supérieure à 15%.

La teneur en acide ajouté au malaxage avant la mise en forme est inférieure à 30%, de préférence comprise entre 0,5 et 20% poids de la masse anhydre en silice et alumine engagée dans la synthèse. L'extrusion peut être réalisée par n'importe quel outil conventionnel, disponible commercialement. La pâte issue du malaxage est extrudée à travers une filière, par exemple à l'aide d'un piston ou d'une monovis ou double vis d'extrusion. Cette étape d'extrusion peut être réalisée par toute méthode connue de l'Homme de métier. Les extrudés du catalyseur d'oligomérisation utilisé dans le procédé de l'invention ont généralement une résistance à l'écrasement d'au moins 70 N/cm et de manière préférée supérieure ou égale à 100 N/cm.

Par ailleurs, ledit catalyseur d'oligomérisation mis en oeuvre dans le procédé selon la présente invention peut avoir été traité ainsi qu'il est bien connu de l'Homme du métier par des additifs pour faciliter la mise en forme et/ou améliorer les propriétés mécaniques finales dudit catalyseur à base de silice-alumine. A titre d'exemple d'additifs, on peut citer notamment la cellulose, la carboxyméthyl-cellulose, la carboxyéthyl-cellulose, les gommes xanthaniques, des agents tensio-actifs, des agents floculants comme les polyacrylamides, le noir de carbone, les amidons, l'acide stéarique, l'alcool polyacrylique, l'alcool polyvinylique, des biopolymères, le glucose, les polyéthylènes glycols, etc.

Le contrôle de la porosité caractéristique du catalyseur d'oligomérisation utilisé dans le procédé de l'invention est opéré partiellement lors de cette étape de mise en forme des particules de catalyseur.

### Séchage et calcination du catalyseur

Pour la préparation du catalyseur d'oligomérisation, une ou plusieurs étapes de séchage et une ou plusieurs étapes de calcination sont réalisées lors de la mise en oeuvre du procédé de préparation du catalyseur d'oligomérisation.

Le séchage est effectué par toute technique connue de l'Homme du métier. Comme mentionné ci-dessus, il est avantageux de réaliser au moins une calcination du solide à base de silice-alumine issu de l'étape a) du procédé de préparation du catalyseur d'oligomérisation, avant la réalisation de l'étape b) de ce même procédé de préparation ou bien de réaliser cette calcination à toute autre étape intermédiaire conduisant au solide à base de silice-alumine de l'étape a) du procédé de préparation du catalyseur décrit plus haut dans la présente description. Il est généralement préférable de calciner en présence d'oxygène moléculaire, par exemple en effectuant un balayage d'air, à une température inférieure ou égale à 1100°C. Ce traitement par exemple peut être effectué en lit traversé, en lit léché ou en atmosphère statique. Par exemple, le four utilisé peut être un four rotatif tournant ou un four vertical à couches traversées radiales. Les conditions de calcination, température et durée, dépendent principalement de la température maximale d'utilisation du catalyseur, les conditions préférées de calcination se situant entre plus d'une heure à 200°C et moins d'une heure à 1100°C. La calcination peut être opérée en présence de vapeur d'eau. La calcination finale peut être éventuellement effectuée en présence d'une vapeur acide ou basique. Par exemple, la calcination peut être réalisée sous pression partielle d'ammoniaque.

Selon un mode de réalisation très préféré du procédé de préparation du catalyseur d'oligomérisation tel que décrit ci-dessus, on procède d'abord à la mise en oeuvre du mélange visé à ladite étape a) de manière à obtenir le solide constitué de silice-alumine précurseur du catalyseur d'oligomérisation. Puis ledit solide est mis en forme sous forme d'extrudés de la manière décrite ci-dessus. Lesdits extrudés sont ensuite séchés et calcinés. Enfin, ils sont soumis au traitement hydrothermal conformément à l'étape b) du procédé de préparation du catalyseur d'oligomérisation, de préférence ledit traitement est une calcination sous air humide.

### Description du procédé d'oligomérisation

Le procédé selon l'invention est un procédé d'oligomérisation des oléfines permettant la production de carburant, par exemple la production d'essence et/ou de kérosène à partir de charges oléfiniques légères contenant entre 2 et 8 atomes de carbone, et en particulier à partir de charges oléfiniques légères contenant une forte proportion de propylène et/ou de butènes et/ou de pentènes utilisant un catalyseur d'oligomérisation à base de silice-alumine à teneur réduite en macropores. De préférence, ledit catalyseur est constitué uniquement de silice-alumine.

### Charges

Les oléfines présentes dans la charge hydrocarbonée oléfinique peuvent provenir par exemple d'une unité de craquage catalytique et/ou d'une unité de vapocraquage, et/ou d'une unité de déshydrogénation de paraffines et/ou d'une unité de déshydratation polymérisante de méthanol en eau et oléfines légères et/ou de toutes autres sources conduisant à la production d'oléfines légères.

La charge hydrocarbonée oléfinique envoyée dans le réacteur d'oligomérisation utilisé pour la mise en oeuvre du procédé de l'invention, contenant le catalyseur préparé selon le procédé décrit ci-dessus, est de préférence débarrassée d'impuretés, telles que par exemple l'eau, les dérivés soufrés, les dérivés azotés basiques, avant d'être introduite dans le réacteur d'oligomérisation.

La charge hydrocarbonée oléfinique peut être une coupe C4 oléfinique, qui comprend habituellement à plus de 90% poids de l'isobutane, du n-butane, du 1-butène, des 2-butènes, de l'isobutène et éventuellement une petite quantité de butadiène. Le butadiène est généralement éliminé en amont de l'oligomérisation par un procédé d'hydrogénation sélective.

La charge hydrocarbonée oléfinique peut être également une coupe C3-C4 oléfinique. La composition de la coupe C3-C4 oléfinique est très variable selon sa provenance. Elle peut comprendre entre environ 20 et 50% poids de propylène et propane, entre environ 50 et 80% poids de l'isobutane, du n-butane, du 1-butène, des 2-butènes, de l'isobutène, et éventuellement une petite quantité de butadiène. Le butadiène est généralement éliminé en amont de l'oligomérisation par un procédé d'hydrogénation sélective.

La charge hydrocarbonée oléfinique peut encore être une coupe C3 oléfinique. Elle comprend habituellement au moins 90% poids de propylène et de propane.

La charge hydrocarbonée oléfinique peut être aussi une coupe C5 oléfinique. La composition de la coupe C5 oléfinique est très variable selon sa provenance. Elle comprend avantageusement entre 30 et 80% poids de C5 oléfinique, entre 1 et 20% poids de C6 oléfinique et entre 1 et 10 % poids de C4 oléfinique.

Conformément à l'invention, l'exothermicité de la réaction d'oligomérisation peut être gérée par un recyclage d'au moins une partie de l'effluent non-converti, qui contient en particulier les paraffines qui n'ont pas été transformées lors de la réaction, vers le réacteur d'oligomérisation, et/ou par dilution de la charge par un ajout de paraffines provenant d'une autre source, lesdites paraffines étant de même poids moléculaire et/ou plus lourdes que la charge oléfinique, lesdites paraffines étant aliphatiques ou cycliques.

Dans tous les cas de procédés conduisant à la formation d'essence et/ou de kérosène, et/ou plus généralement d'une coupe oléfinique avec un point d'ébullition commençant à une température supérieure à 150°C, les coupes oléfiniques obtenues en sortie du procédé peuvent éventuellement être hydrogénées, partiellement ou totalement.

### Modes de réalisation du procédé d'oligomérisation de l'invention

### Premier mode de réalisation : oligomérisation sélective

Selon ledit premier mode de réalisation, on met en contact une coupe C4 oléfinique avec le catalyseur comprenant une silice - alumine et préparé selon le procédé tel que décrit dans la présente description de manière à limiter la conversion globale des n-butènes à moins de 10% poids, de manière préférée à moins de 5% poids, alors que plus de 90% poids de la quantité d'isobutène est convertie, de préférence plus de 95% poids. L'isobutène est converti à plus de 90% poids en dimères et trimères. Ensuite l'effluent d'oligomérisation est soumis à une distillation de telle sorte qu'une des fractions récupérées (effluent léger) contient à plus de 90% poids du butane, de l'isobutane et les butènes qui n'ont pas réagi lors de l'oligomérisation, une partie au moins de cette fraction alimentant ensuite par exemple une unité d'alkylation ou une unité d'hydratation, alors que l'autre fraction constituée des oligomères obtenus est utilisée comme base essence, éventuellement après hydrogénation partielle ou totale.

Le mode de réalisation du procédé d'oligomérisation décrit ci-dessus correspond au mode de réalisation dit d"oligomérisation sélective" dans lequel l'isobutène est majoritairement converti.

Conformément audit premier mode de réalisation du procédé d'oligomérisation de l'invention, la réaction d'oligomérisation est effectuée à une température comprise entre 30 et 300°C, sous une pression comprise entre 0,1 et 20 MPa et le volume de charge hydrocarbonée oléfinique envoyé par volume de catalyseur et par heure est compris entre 0,05 et 5 h⁻¹. De préférence la température est comprise entre 40 et 160°C, et la pression entre 2 et 7 MPa, de façon à s'assurer que la réaction s'effectue en phase liquide, ou au moins en phase homogène (c'est-à-dire entièrement en phase liquide ou entièrement en phase gazeuse), et le volume de charge hydrocarbonée oléfinique envoyé par volume de catalyseur et par heure se situe de préférence entre 0,1 et 2,5 h⁻¹.

La technologie du réacteur d'oligomérisation peut être un lit fixe, un lit fluidisé ou un lit circulant. La technologie préférée est une mise en oeuvre en lit fixe.

De manière préférée, les oligomères ainsi obtenus sont réinjectés dans un réacteur d'oligomérisation supplémentaire contenant par exemple le catalyseur d'oligomérisation comprenant une silice - alumine tel que précédemment décrit, de façon à augmenter la longueur de chaîne des oligomères et atteindre ainsi la coupe kérosène, ou plus généralement une coupe oléfinique avec un point d'ébullition commençant à une température supérieure à 150°C.

De manière avantageuse, l'effluent léger d'oligomérisation, c'est-à-dire la coupe C4, peut être introduit dans un réacteur d'hydroisomérisation visant à hydroisomériser une partie du 1-butène non-converti en 2-butène, de façon à se rapprocher de l'équilibre thermodynamique. Les autres constituants de l'effluent ne sont alors pas convertis de façon significative durant l'étape d'hydroisomérisation. La conversion du 1-butène en 2-butène est très utile si la fraction C4 ainsi obtenue en sortie du réacteur d'hydroisomérisation peut ensuite être introduite dans un réacteur d'alkylation aliphatique sur acide fluorhydrique, les produits obtenus par alkylation du 2-butène avec l'isobutane ayant un meilleur indice d'octane que l'alkylat obtenu à partir du 1-butène.

Étant donné la forte exothermicité de la réaction d'oligomérisation, la quantité d'isobutène dans la charge hydrocarbonée alimentant le réacteur d'oligomérisation est de préférence inférieure à 35% poids, de manière encore plus préférée inférieure à 15% poids, ladite quantité étant éventuellement obtenue en diluant la charge, par exemple avec du butane ou de l'isobutane ou du raffinat de l'unité d'oligomérisation.

### Deuxième mode de réalisation

Selon ledit deuxième mode de réalisation, on met en contact une coupe C4 oléfinique ou une coupe C3-C4 oléfinique avec le catalyseur d'oligomérisation décrit dans la présente description de manière à ce qu'une partie des butènes contenus dans la charge hydrocarbonée soit convertie en dimères ou trimères, utilisés ensuite comme base essence. Conformément audit deuxième mode de réalisation du procédé de l'invention, moins de 80% poids des butènes sont convertis et au moins 80% poids, de préférence au moins 90% poids de l'isobutène sont convertis. Ce procédé permet de produire une quantité maximale d'essence tout en minimisant la quantité de kérosène formé.

Dans le réacteur d'oligomérisation utilisé pour la mise en oeuvre dudit deuxième mode de réalisation, la température se situe entre 40 et 250°C, de préférence entre 50 et 200°C, et la pression se situe entre 0,1 et 10 MPa, de préférence entre 0,1 et 6 MPa, et la quantité de charge hydrocarbonée envoyée par volume de catalyseur et par heure se situe entre 0,05 et 5 h⁻¹, de préférence entre 0,1 et 2,5 h⁻¹. La technologie du réacteur peut être un lit fixe, un lit fluidisé ou un lit circulant. La technologie préférée met en oeuvre un lit fixe.

Une variante dudit deuxième mode de réalisation du procédé de l'invention consiste à utiliser comme charge une charge oléfinique dans laquelle l'isobutène a été au préalable éliminé en partie ou totalement, par exemple en utilisant en amont de l'unité d'oligomérisation une unité d'éthérification en faisant réagir sélectivement l'isobutène avec un alcool, par exemple le méthanol ou l'éthanol, sans convertir le n-butène, ou bien en utilisant en amont de l'unité d'oligomérisation une unité d'oligomérisation sélective telle que celle décrite plus haut dans ledit premier mode de réalisation. Les oligomères produits présentent alors moins de branchements que ceux obtenus par traitement de la coupe complète incluant l'isobutène.

### Troisième mode de réalisation

Un troisième mode de réalisation du procédé selon l'invention consiste à soumettre une coupe C4 oléfinique, contenant éventuellement des traces de propylène, à une oligomérisation de telle sorte que la majeure partie des butènes contenus dans la charge soit convertie en dimères ou trimères, utilisés ensuite comme base essence. Conformément audit troisième mode de réalisation du procédé de l'invention, au moins 90% poids des 1-butène, au moins 80% poids des 2-butènes, au moins 97% poids de l'isobutène et au moins 80% poids du propylène sont convertis. Ledit troisième mode de réalisation du procédé de l'invention permet de produire une quantité maximale d'essence sans fabriquer de kérosène. La coupe C4 oléfinique comprend habituellement de l'isobutane, du n-butane, du 1-butène, du 2-butène, de l'isobutène et éventuellement une petite quantité de butadiène. Le butadiène est généralement éliminé en amont de l'oligomérisation par un procédé d'hydrogénation sélective, pour éviter des réactions de polymérisation qui rendraient le catalyseur inactif.

Ledit procédé mis en oeuvre conformément audit troisième mode de réalisation comprend les étapes suivantes :
- une première étape d'oligomérisation : on traite une coupe C4 oléfinique, dans un premier réacteur d'oligomérisation dans lequel la conversion globale des n-butènes contenus dans la charge est inférieure à 45% poids et la conversion de l'isobutène est supérieure à 80% poids, de préférence supérieure à 85% poids, les oligomères obtenus étant des dimères et trimères à plus de 80% poids,
- on envoie l'effluent de la première étape d'oligomérisation dans une colonne de fractionnement de façon à récupérer une première fraction contenant l'isobutène et les n-butènes non-convertis et une deuxième fraction consistant à 90% poids en dimères et trimères de la réaction d'oligomérisation,
- une deuxième étape d'oligomérisation : ladite première fraction récupérée est introduite dans un deuxième réacteur d'oligomérisation dans lequel les oléfines sont converties en grande partie en dimères et trimères, c'est-à-dire qu'au moins 50% poids des n-butènes sont convertis, de préférence au moins 75% poids du 1-butène et au moins 55% poids du 2-butène sont convertis et,
- on envoie l'effluent de la deuxième étape d'oligomérisation dans la colonne de fractionnement associée au premier réacteur d'oligomérisation ou dans une deuxième colonne pour séparer l'essence ou le kérosène des composés en C4 non-convertis.

Dans les réacteurs d'oligomérisation, la température se situe entre 40 et 250°C, de préférence entre 45 et 200°C, et la pression se situe entre 0,1 et 10 MPa, de préférence entre 0,1 et 6 MPa, et la quantité de charge hydrocarbonée envoyée par volume de catalyseur et par heure se situe entre 0,05 et 5 h⁻¹, de préférence entre 0,1 et 2,5 h⁻¹. La technologie du réacteur peut être un lit fixe, un lit fluidisé ou un lit circulant. De préférence la technologie est un lit fixe.

De préférence, dans le deuxième réacteur d'oligomérisation, les conditions opératoires sont plus sévères que dans le premier réacteur.

Ledit troisième mode de réalisation du procédé de l'invention peut être appliqué à une charge C3-C4 oléfinique.

### Quatrième mode de réalisation

Selon ledit quatrième mode de réalisation, on met une coupe C4 oléfinique ou une coupe C3-C4 oléfinique en contact avec le catalyseur d'oligomérisation tel que décrit dans la présente description de telle sorte que la majeure partie des butènes contenus dans la charge soit convertie, de façon à former une base essence et une base kérosène. Conformément audit quatrième mode de réalisation du procédé de l'invention, au moins 90% poids des 1-butène, au moins 80% poids des 2-butènes et au moins 97% poids de l'isobutène sont convertis. La coupe C4 oléfinique comprend habituellement essentiellement de l'isobutane, du n-butane, du 1-butène, du 2-butène, de l'isobutène et éventuellement une petite quantité de butadiène. La coupe C3-C4 oléfinique comprend en plus du propane et du propylène dans les proportions données plus haut dans la présente description.

Dans le réacteur d'oligomérisation, la température se situe entre 60 et 250°C, de préférence entre 100 et 200°C, et la pression se situe entre 0,1 et 10 MPa, de préférence entre 0,1 et 6 MPa, et la quantité de charge hydrocarbonée envoyée par volume de catalyseur et par heure se situe entre 0,05 et 5 h⁻¹, de préférence entre 1 et 2,5 h⁻¹. La technologie du réacteur peut être un lit fixe, un lit fluidisé ou un lit circulant. De préférence la technologie est un lit fixe.

### Cinquième mode de réalisation

Selon ledit cinquième mode de réalisation, on met une coupe C3 oléfinique en contact avec ledit catalyseur d'oligomérisation décrit dans la présente description de telle sorte que la majeure partie du propylène contenu dans la charge soit convertie, c'est à dire qu'au moins 80% poids du propylène contenu dans la charge sont convertis, de façon à former une base essence et une base kérosène. La coupe C3 oléfinique comprend habituellement au moins 90% poids de propylène et de propane.

La réaction d'oligomérisation est effectuée à une température entre 30 et 300°C, sous une pression entre environ 0,1 et 20 MPa et le volume de charge hydrocarbonée envoyé par volume de catalyseur et par heure se situe entre 0,05 et 5 h⁻¹. De préférence la température se situe entre 40 et 160°C, et la pression entre 2 et 7 MPa, le volume de charge hydrocarbonée envoyé par volume de catalyseur et par heure se situe de préférence entre 0,1 et 2,5 h-1. La technologie du réacteur peut être un lit fixe, un lit fluidisé ou un lit circulant. La technologie préférée est une mise en oeuvre en lit fixe.

### Techniques de caractérisation

Le catalyseur à base d'une silice-alumine, utilisé dans le procédé d'oligomérisation de l'invention, est caractérisé par plusieurs techniques d'analyses et notamment par Diffraction des Rayons X aux grands angles (DRX), par isotherme d'adsorption d'azote, par intrusion au porosimètre à mercure, par Microscopie Électronique à Transmission (MET) éventuellement couplée à une analyse par spectrométrie de rayons X à sélection d'énergie (EDX), par Rayonnement Magnétique Nucléaire du solide de l'atome d'aluminium (RMN MAS ²⁷Al), par spectroscopie infrarouge (IR) et par Fluorescence X (FX) ou Absorption Atomique (AA). La densité du catalyseur utilisé dans le procédé de l'invention est également évaluée.

La technique de Diffraction des Rayons X aux grands angles (valeurs de l'angle 2θ comprises entre 5 et 70°) permet de caractériser un solide cristallisé défini par la répétition d'un motif unitaire ou maille élémentaire à l'échelle moléculaire. Dans l'exposé qui suit, l'analyse des rayons X est réalisée sur poudre avec un diffractomètre opérant en réflexion et équipé d'un monochromateur arrière en utilisant la radiation du cuivre (longueur d'onde de 1,5406 Å). Les pics habituellement observés sur les diffractogrammes correspondants à une valeur donnée de l'angle 2θ sont associés aux distances inter-réticulaires d₍ₕₖₗ₎ caractéristiques de la (des) symétrie(s) structurale(s) du catalyseur, ((hkl) étant les indices de Miller du réseau réciproque) par la relation de Bragg : 2 d ₍ₕₖₗ₎ * sin (9) = n * λ. Cette indexation permet alors la détermination des paramètres de maille (abc) du réseau direct. En particulier, les 2 pics les plus intenses sont situés à une position correspondant à un d compris entre 1,39 et 1,40 Å et un d compris entre 1,97 Å et 2,00 Å. Par alumine gamma, on entend dans la suite du texte, entre autres et par exemple, une alumine comprise dans le groupe composé des alumines gamma cubique, gamma pseudo-cubique, gamma tétragonale, gamma mal ou peu cristallisée, gamma grande surface, gamma basse surface, gamma issue de grosse boehmite, gamma issue de boehmite cristallisée, gamma issue de boehmite peu ou mal cristallisée, gamma issue d'un mélange de boehmite cristallisée et d'un gel amorphe, gamma issue d'un gel amorphe, gamma en évolution vers delta. Pour les positions des pics de diffraction des alumines êta, delta et thêta, il est possible de se référer à l'article de B. C. Lippens, J. J. Steggerda, dans "Physical and Chemical Aspects of Adsorbents and Catalysts", E. G. Linsen (Ed.), Academic Press, London, 1970, 171. Pour le catalyseur utilisé dans le procédé selon l'invention, le diagramme de diffraction X met en évidence un pic large caractéristique de la présence de silice amorphe. Par ailleurs, dans l'ensemble du texte qui suit, le composé aluminique peut contenir une fraction amorphe difficilement détectable par les techniques de DRX. Il sera donc sous-entendu par la suite que les composés aluminiques utilisés ou décrits dans le texte peuvent contenir une fraction amorphe ou mal cristallisée.

L'analyse isotherme d'adsorption d'azote correspondant à l'adsorption physique de molécules d'azote dans la porosité du catalyseur via une augmentation progressive de la pression à température constante renseigne sur les caractéristiques texturales (diamètre de pores, type de porosité, surface spécifique) particulières du catalyseur d'oligomérisation utilisé dans le procédé selon l'invention. En particulier, elle permet d'accéder à la surface spécifique et à la distribution mésoporeuse dudit catalyseur. On entend par surface spécifique, la surface spécifique B.E.T. (S_{BET} en m²/g) déterminée par adsorption d'azote conformément à la norme ASTM D 3663-78 établie à partir de la méthode BRUNAUER-EMMETT-TELLER décrite dans le périodique "The Journal of American Society", 1938, 60, 309. La distribution poreuse représentative d'une population de mésopores centrée dans une gamme de 1,5 à 50 nm est déterminée par le modèle Barrett-Joyner-Halenda (BJH). L'isotherme d'adsorption - désorption d'azote selon le modèle BJH est décrit dans le périodique *"*The Journal of American Society", 1951, 73, 373, écrit par E. P. Barrett, L. G. Joyner et P. P. Halenda. Dans l'exposé qui suit, le "volume adsorption azote du catalyseur" correspond au volume mesuré pour P/P₀ = 0,99, pression pour laquelle il est admis que l'azote a rempli tous les pores. Pour finir, la dénomination "surface adsorption" se rapporte à la surface mesurée sur la branche de l'isotherme d'adsorption. On se reportera par exemple à l'article de A. Lecloux dans "Mémoires de la Société Royale des Sciences de Liège", 1971, 6ème série, Tome I, fasc.4, 169.

Dans l'exposé qui suit, le "volume mercure du catalyseur" correspond au volume mesuré par intrusion au porosimètre à mercure selon la norme ASTM D4284-83 à une pression maximale de 4000 bar, utilisant une tension de surface de 484 dyne/cm et un angle de contact pour le catalyseur d'oligomérisation comprenant une silice-alumine amorphe de 140°. Le diamètre moyen mercure est défini comme étant un diamètre tel que tous les pores de taille inférieure à ce diamètre constituent 50% du volume poreux (V_{Hg}), dans un intervalle compris entre 36 Å et 1000 Å. L'angle de mouillage a été pris égal à 140° en suivant les recommandations de l'ouvrage "Techniques de l'ingénieur, traité analyse et caractérisation", 1050, de J. Charpin et B. Rasneur. Afin d'obtenir une meilleure précision, la valeur du volume mercure en ml/g donnée dans le texte qui suit correspond à la valeur du volume mercure total en ml/g mesurée sur l'échantillon moins la valeur du volume mercure en ml/g mesurée sur le même échantillon pour une pression correspondant à 30 psi (environ 2 bar). Afin de mieux caractériser la distribution poreuse résultante de l'analyse par intrusion au mercure, les critères de distribution poreuse suivants sont définis : le volume V2 correspond au volume contenu dans les pores de diamètre supérieur ou égal au diamètre moyen moins 30 Å et inférieur ou égal au diamètre moyen plus 30 Å et le volume V3 correspond au volume contenu dans les pores de diamètre supérieur ou égal au diamètre moyen plus 30 Å.

L'analyse par Microscopie Electronique par Transmission (MET) est une technique également largement utilisée pour caractériser le catalyseur d'oligomérisation à base de silice-alumine utilisé dans le procédé de l'invention. Celle-ci permet la formation d'une image du solide étudié, les contrastes observés étant caractéristiques de l'organisation structurale, de la texture, de la morphologie ou bien de la composition des particules observées, la résolution de la technique atteignant au maximum 0,2 nm. Pour cela un microscope électronique (du type Jeol 2010 ou Philips Tecnai20F éventuellement avec balayage) équipé d'un spectromètre de rayons X à sélection d'énergie (EDX) (par exemple un Tracor ou un Edax) est utilisé. Le détecteur EDX doit permettre la détection des éléments légers. L'association de ces deux outils, MET et EDX, permet de combiner l'imagerie et l'analyse chimique locale avec une bonne résolution spatiale. Pour ce type d'analyse, les échantillons sont finement broyés à sec dans un mortier. La poudre est ensuite incluse dans de la résine pour réaliser des coupes ultrafines d'épaisseur 70 nm environ. Ces coupes sont recueillies sur des grilles de cuivre recouvertes d'un film de carbone amorphe à trous servant de support. Elles sont ensuite introduites dans le microscope pour observation et analyse sous vide secondaire. En imagerie, les zones d'échantillon des zones de résine se distinguent aisément. Un certain nombre d'analyses sont ensuite effectuées, 10 au minimum, de préférence entre 15 et 30, sur différentes zones de l'échantillon. La taille du faisceau électronique pour l'analyse des zones (déterminant approximativement la taille des zones analysées) est de 50 nm de diamètre au maximum, de préférence de 20 nm, de manière encore plus préférée de 10, 5, 2 ou 1 nm de diamètre. En mode balayé, la zone analysée sera fonction de la taille de la zone balayée et non plus de la taille du faisceau généralement réduit. Le traitement semi-quantitatif des spectres X recueillis à l'aide du spectromètre EDX permet d'obtenir la concentration relative des éléments Al et Si (en % atomique) et le rapport atomique Si/Al pour chacune des zones analysées. La moyenne Si/Alₘ et l'écart type σ de cet ensemble de mesures peuvent alors être calculés. Dans les exemples non limitatifs de l'exposé qui suit de l'invention, la sonde de 50 nm est la sonde utilisée pour caractériser le catalyseur d'oligomérisation à base de silice-alumine utilisé dans le procédé de l'invention sauf mention contraire.

Le catalyseur d'oligomérisation comprenant une silice-alumine et utilisé dans le procédé de l'invention a été analysé par RMN MAS du solide de ²⁷Al sur un spectromètre de la firme Brüker de type MSL 400, en sonde 4 mm. La vitesse de rotation des échantillons est de l'ordre de 11 kHz. Potentiellement, la RMN de l'aluminium permet de distinguer trois types d'aluminium dont les déplacements chimiques sont reportés ci-après :
- entre 100 et 40 ppm, aluminiums de type tétracoordinés, notés Al_{IV},
- entre 40 et 20 ppm, aluminiums de type pentacoordinés, notés Al_{V},
- entre 20 et - 100 ppm, aluminiums de type hexacoordinés, notés Al_{VI}.

L'atome d'aluminium est un noyau quadripolaire. Dans certaines conditions d'analyse (champs de radiofréquence faible : 30 kHz, angle d'impulsion faible : π/2 et échantillon saturé en eau), la technique de RMN de rotation à l'angle magique (MAS) est une technique quantitative. La décomposition des spectres RMN MAS permet d'accéder directement à la quantité des différentes espèces. Le spectre est calé en déplacement chimique par rapport à une solution 1 M de nitrate d'aluminium. Le signal d'aluminium est à zéro ppm. On a choisi d'intégrer les signaux entre 100 et 20 ppm pour les Al_{IV} et Al_{V}, ce qui correspond à l'aire 1, et entre 20 et -100 ppm pour Al_{VI}, ce qui correspond à l'aire 2. Dans l'exposé qui suit de l'invention, on entend par proportion des Al_{VI} octaédriques le rapport suivant : aire 2/(aire 1 + aire 2).

L'acidité du catalyseur d'oligomérisation est mesurée par spectroscopie infrarouge. Les spectres IR sont enregistrés sur un interféromètre Nicolet de type Nexus-670 sous une résolution de 4 cm⁻¹ avec une apodisation de type Happ-Gensel. L'échantillon (20 mg) est pressé sous la forme d'une pastille auto-supportée et placé dans une cellule d'analyse in situ (25 à 550°C, four déporté du faisceau IR, vide secondaire de 10⁻⁶ mbar). Le diamètre de la pastille est de 16 mm. L'échantillon est prétraité de la façon suivante afin d'éliminer l'eau physisorbée et de déshydroxyler partiellement la surface du catalyseur pour avoir une image représentative de l'acidité du catalyseur en fonctionnement :
- montée en température de 25 à 300°C en 3 heures,
- palier de 10 heures à 300°C,
- descente de température de 300 à 25°C en 3 heures.

La sonde basique (pyridine) est ensuite adsorbée à pression saturante à 25°C puis thermodésorbée selon les paliers suivants :
- 25°C pendant 2 heures sous vide secondaire,
- 100°C pendant 1 heure sous vide secondaire,
- 200°C pendant 1 heure sous vide secondaire,
- 300°C pendant 1 heure sous vide secondaire.

Un spectre est enregistré à 25°C à la fin du prétraitement et à chaque palier de désorption en mode transmission avec un temps d'accumulation de 100 s. Les spectres sont ramenés à isomasse (donc supposée à isoépaisseur) (20 mg exactement). Le nombre de sites acides de Lewis est proportionnel à la surface du pic dont le maximum se situe vers 1450 cm⁻¹, tout épaulement étant inclus. Le nombre de sites acides de Brönsted est proportionnel à la surface du pic dont le maximum se situe vers 1545 cm⁻¹. Le rapport du nombre de sites acides de Brönsted /nombre de sites acides de Lewis (B/L) est estimé égal au rapport des surfaces de deux pics décrits ci-dessus. On utilise généralement la surface des pics à 25°C. Ce rapport B/L est, de manière générale, calculé à partir du spectre enregistré à 25°C après adsorption de la pyridine et du palier de 2h sous vide secondaire.

La composition globale du catalyseur d'oligomérisation utilisé dans le procédé de l'invention, et en particulier la teneur en élément sodium, peut être déterminée par Fluorescence X (FX) sur ledit catalyseur à l'état pulvérulent ou par Absorption Atomique (AA) après attaque acide dudit catalyseur.

La densité de remplissage tassée (DRT) est mesurée, comme cela est décrit dans l'ouvrage *"*Applied Heterogeneous Catalysis" de J. F. Le Page, J. Cosyns, P. Courty, E. Freund, J. -P. Franck, Y. Jacquin, B. Juguin, C. Marcilly, G. Martino, J. Miquel, R. Montarnal, A. Sugier, H. Van Landeghem, Technip, Paris, 1987, chapitre 6.2.4, pages 167-168. Un cylindre gradué de dimensions acceptables est rempli par additions successives et, entre deux additions successives, le catalyseur est tassé en secouant le cylindre jusqu'à atteindre un volume constant. Cette mesure est généralement réalisée sur 1000 cm³ de catalyseur tassé dans un cylindre dont le ratio hauteur sur diamètre est proche de 5:1. Cette mesure peut être, de manière préférée, réalisée sur des appareils automatisés tels que Autotap® commercialisé par Quantachrome®.

Les exemples suivants illustrent la présente invention sans toutefois en limiter la portée.

### Exemple 1 : préparation et mise en forme d'un catalyseur constitué d'une silice-alumine SA1 (conforme à l'invention)

Le catalyseur constitué de silice-alumine SA1 conforme à l'invention est préparé en procédant d'abord au mélange d'une combinaison formée d'un composé aluminique et d'un composé silicique, l'un et l'autre étant totalement solubles dans ledit mélange (le rapport R décrit plus haut dans la présente description étant égal à 0,92), avec un composé aluminique partiellement soluble en milieu acide. Pour la préparation de ladite combinaison, on opère comme suit : dans un premier temps, une solution d'acide sulfurique 30% est ajoutée à une solution de silicate de sodium. La quantité d'H₂SO₄ est définie de manière à travailler à un taux de neutralisation fixé. L'addition se fait en deux minutes sous une agitation de 600 tours/minutes. La température de synthèse est de 60°C. La durée de mûrissement a été fixée à 30 minutes. L'agitation est maintenue à 600 tours/minutes, la température est celle de l'étape précédente. Puis, on ajoute Al₂(SO₄)₃ (500 ml), la concentration est fixée par la teneur en alumine désirée. Le pH n'est pas régulé et est fixé par la teneur en alumine désirée. L'ajout se fait en 10 minutes. L'agitation est toujours fixée à 600 tours/minute, la température est la même que celle des étapes précédentes. Puis, on ajoute l'ammoniaque. Le gel obtenu est filtré par déplacement. Le lavage se fait à l'eau à 60°C, 3 kg d'eau par kg de solide contenu dans le gel. Puis, un échange au nitrate d'ammonium NH₄NO₃(138,5 g/l) à 60°C et 1,5 I par kg de solide contenu dans le gel est effectué. Enfin, un lavage supplémentaire à l'eau à 60°C est fait par déplacement, 3 kg d'eau par kg de solide contenu dans le gel. Le gel issu de cette étape est mélangé avec de la poudre de boehmite Pural (composé aluminique partiellement soluble) de façon à ce que la composition finale du solide mixte en produit anhydre soit, à ce stade de la synthèse, égale à 50% Al₂O₃-50% SiO₂. Le malaxage se fait sur un malaxeur bras en Z. L'extrusion est réalisée par passage de la pâte au travers d'une filière munie d'orifices de diamètre 1,4 mm. Les extrudés ainsi obtenus sont séchés à 150°C, calcinés à 550°C, puis calcinés à 700°C en présence de 400 g d'eau par kg d'air sec pendant une durée de 6 heures.Les caractéristiques du catalyseur constitué de silice-alumine sont les suivantes :
- la composition du support est 49% Al₂O₃- 51 % SiO₂,
- la surface BET est de 284 m²/g,
- le volume poreux total, mesuré par Intrusion au porosimètre à mercure, est de 0,85 ml/g,
- le diamètre poreux moyen, mesuré Intrusion au porosimètre à mercure, est de 110 Å,
- le rapport entre le volume V2, mesuré par Intrusion au porosimètre à mercure, compris entre le Dmoyen - 30 Å et le Dmoyen + 30 Å sur le volume mercure total est de 0,85,
- le volume V3, mesuré par Intrusion au porosimètre à mercure, compris dans les pores de diamètre supérieur à Dmoyen + 30 Å est de 0,05 ml/g,
- le rapport entre la surface adsorption et la surface BET est de 0,76,
- le volume poreux, mesuré par Intrusion au porosimètre à mercure, compris dans les pores de diamètre supérieur à 160 Å est de 0,04 ml/g,
- le volume poreux, mesuré par Intrusion au porosimètre à mercure, compris dans les pores de diamètre supérieur à 200 Å est 0,03 ml/g,
- le volume poreux, mesuré par Intrusion au porosimètre à mercure, est compris dans les pores de diamètre supérieur à 500 Å est de 0,009 ml/g,
- la densité de remplissage tassée du support est de 0,47 g/cm³,
- le diagramme de diffraction X contient les raies principales caractéristiques de l'alumine gamma et il contient notamment les pics à un d compris entre 1,39 et 1,40 Å et à un d compris entre 1,97 Å et 2,00 Å.
- le rapport B/L du catalyseur est de 1,
- la teneur en sodium atomique est de 300 +/- 20 ppm. La teneur en soufre atomique est de 2500 ppm,
- le spectre RMN MAS du solide de ²⁷Al du catalyseur montre deux massifs de pics distincts. Un premier type d'aluminium dont le maximum résonne vers 10 ppm s'étend entre -100 et 20

ppm. La position du maximum suggère que ces espèces sont essentiellement de type Al_{VI} (octaédrique). Un deuxième type d'aluminium majoritaire dont le maximum résonne vers 60 ppm s'étend entre 20 et 100 ppm. L'espèce prédominante de ce massif correspondrait aux atomes d'Al_{IV} (tétraédrique). - le catalyseur contient une seule zone silico-aluminique avec un rapport Si/Al déterminé par microsonde en MET de 1,1.

### Exemple 2 : préparation et mise en forme d'un catalyseur constitué d'une silice-alumine SA2 (non - conforme à l'invention)

La poudre d'hydroxyde d'aluminium a été préparée selon le procédé décrit dans la demande de brevet WO 00/01 617. La taille des particules moyenne des particules d'hydroxyde d'aluminium mesurée par granulométrie laser est de 40 microns. Cette poudre est mélangée à un sol de silice préparé par échange sur résine décationisante, puis filtrée sur résine de porosité 2. Les concentrations en sol de silice et en poudre d'hydroxyde d'aluminium sont ajustées de manière à obtenir une composition finale de 70% Al₂O₃ et de 30% SiO₂. La mise en forme est réalisée en présence de 15% d'acide nitrique par rapport au produit anhydre. Le malaxage se fait sur un malaxeur bras en Z. L'extrusion est réalisée par passage de la pâte au travers d'une filière munie d'orifices de diamètre 1,4 mm. Les extrudés ainsi obtenus sont séchés à 150°C, puis calcinés à 550°C.

Les caractéristiques du catalyseur sont les suivantes :
- la composition du catalyseur en silice-alumine est de 85,3 % Al₂O₃ et de 14,7 % SiO₂,
- la surface BET est de 430 m²/g,
- le volume poreux total, mesuré par adsorption d'azote, est de 0,24 ml/g,
- le diamètre poreux moyen, mesuré par porosimétrie au mercure, est de 46 Å.
- le rapport entre le volume V2, mesuré par porosimétrie au mercure, compris entre le D_{moyen}
- 30 Å et le D_{moyen} +30 Å sur le volume mercure total est de 0,7,
- le volume V3, mesuré par porosimétrie au mercure, compris dans les pores de diamètre supérieur à D_{moyen} +30 Å est de 0,07 ml/g,
- le volume poreux, mesuré par porosimétrie au mercure, compris dans les pores de diamètre supérieur à 160 Å est de 0,051 ml/g,
- le volume poreux, mesuré par porosimétrie au mercure, compris dans les pores de diamètre supérieur à 200 Å est de 0,047 ml/g,
- le volume poreux, mesuré par porosimétrie au mercure, est compris dans les pores de diamètre supérieur à 500 Å est de 0,03 ml/g,
- le rapport B/L du catalyseur est de 1,1,
- la densité de remplissage tassée du catalyseur est de 0,80 g/cm³,
- le diagramme de diffraction X contient les raies principales caractéristiques de l'alumine gamma et notamment il contient les pics à un d compris entre 1,39 et 1,40 Å et à un d compris entre 1,97 Å et 2,00 Å,
- la teneur en sodium atomique est de 40 +/- 20 ppm. La teneur en soufre atomique est de 200 ppm,
- le spectre RMN MAS du solide de ²⁷Al du catalyseur montre deux massifs de pics distincts. Un premier type d'aluminium dont le maximum résonne vers 10 ppm s'étend entre -100 et 20 ppm. La position du maximum suggère que ces espèces sont essentiellement de type Al_{VI} (octaédrique). Un deuxième type d'aluminium minoritaire dont le maximum résonne vers 60 ppm et qui s'étend entre 20 et 100 ppm. Ce massif peut être décomposé en au moins deux espèces. L'espèce prédominante de ce massif correspondrait aux atomes d'Al_{IV} (tétraédrique),
- le catalyseur contient deux zones silico-aluminiques, les dites zones ayant des rapports Si/Al inférieurs ou supérieurs au rapport Si/Al global déterminé par fluorescence X. L'une des zones a un rapport Si/Al déterminé par MET de 4,35 et l'autre zone a un rapport Si/Al déterminé par MET de 1,75.

### Exemple 3 : préparation et mise en forme d'un catalyseur constitué d'une silice-alumine SA3 (non conforme à l'invention)

Le catalyseur constitué de silice-alumine SA3 (non conforme à l'invention) est préparé en procédant d'abord au mélange d'une combinaison formée d'un composé aluminique et d'un composé silicique, l'un et l'autre étant totalement solubles dans ledit mélange, avec un composé aluminique partiellement soluble en milieu acide. Pour la préparation de ladite combinaison, on opère comme suit : dans un premier temps une solution d'acide sulfurique 30% est ajoutée à une solution de silicate de sodium. La quantité d'H₂SO₄ est définie de manière à travailler à un taux de neutralisation fixé. L'addition se fait en deux minutes sous une agitation de 600 tours/minutes. La température de synthèse est de 60°C. La durée de mûrissement a été fixée à 30 minutes. L'agitation est maintenue à 600 tours/minutes, la température est celle de l'étape précédente. Puis, on ajoute Al₂(SO₄)₃ (500 ml), la concentration est fixée par la teneur en alumine désirée. Le pH n'est pas régulé et est fixé par la teneur en alumine désirée. L'ajout se fait en 10 minutes. L'agitation est toujours fixée à 600 tours/minute, la température est la même que celle des étapes précédentes. Puis, on ajoute l'ammoniaque. Le gel obtenu est filtré par déplacement. Le lavage se fait à l'eau à 60°C, 3 kg d'eau par kg de solide contenu dans le gel. Puis, un échange au nitrate d'ammonium NH₄NO₃(138,5 g/l) à 60°C et 1,5 I par kg de solide contenu dans le gel est effectué. Enfin, un lavage supplémentaire à l'eau à 60°C est fait par déplacement, 3 kg d'eau par kg de solide contenu dans le gel. Le gel issu de cette étape est mélangé avec de la poudre de boehmite Pural (composé aluminique partiellement soluble) de façon à ce que la composition finale du support mixte en produit anhydre soit, à ce stade de la synthèse, égale à 50% Al₂O₃-50% SiO₂. Le malaxage se fait sur un malaxeur bras en Z. L'extrusion est réalisée par passage de la pâte au travers d'une filière munie d'orifices de diamètre 1,4 mm. Les extrudés ainsi obtenus sont séchés à 150°C, calcinés à 550°C, puis calcinés à 700°C en présence de 400 g d'eau par kg d'air sec pendant une durée de 2 heures.

Les caractéristiques du catalyseur constitué de silice-alumine sont les suivantes :
- la composition du support est 49% Al₂O₃- 51% SiO₂,
- la surface BET est de 270 m²/g,
- le volume poreux total, mesuré par Intrusion au porosimètre à mercure, est de 0,55 ml/g,
- le diamètre poreux moyen, mesuré par Intrusion au porosimètre à mercure, est de 7,5 Å,
- le rapport entre le volume V2, mesuré par Intrusion au porosimètre à mercure, compris entre le Dmoyen - 30 Å et le Dmoyen + 30 Å sur le volume mercure total est de 0,85,
- le volume V3, mesuré par Intrusion au porosimètre à mercure, compris dans les pores de diamètre supérieur à Dmoyen + 30 Å est de 0,07 ml/g,
- le rapport entre la surface adsorption et la surface BET est de 0,75,
- le volume poreux, mesuré par Intrusion au porosimètre à mercure, compris dans les pores de diamètre supérieur à 160 Å est de 0,04 ml/g,
- le volume poreux, mesuré par Intrusion au porosimètre à mercure, compris dans les pores de diamètre supérieur à 200 Å est 0,03 ml/g,
- le volume poreux, mesuré par Intrusion au porosimètre à mercure, est compris dans les pores de diamètre supérieur à 500 Å est de 0,009 ml/g,
- la densité de remplissage tassée du support est de 0,63 g/cm³,
- le diagramme de diffraction X contient les raies principales caractéristiques de l'alumine gamma et il contient notamment les pics à un d compris entre 1,39 et 1,40 Å et à un d compris entre 1,97 Å et 2,00 Å.
- le rapport B/L du catalyseur est de 1,
- la teneur en sodium atomique est de 300 +/- 20 ppm. La teneur en soufre atomique est de 2500 ppm,
- le spectre RMN MAS du solide de ²⁷Al du catalyseur montre deux massifs de pics distincts. Un premier type d'aluminium dont le maximum résonne vers 10 ppm s'étend entre -100 et 20 ppm. La position du maximum suggère que ces espèces sont essentiellement de type Al_{VI}, (octaédrique). Un deuxième type d'aluminium majoritaire dont le maximum résonne vers 60 ppm s'étend entre 20 et 100 ppm. L'espèce prédominante de ce massif correspondrait aux atomes d'Al_{VI} (tétraédrique).
- le support contient une seule zone silico-aluminique avec un rapport Si/Al déterminé par microsonde en MET de 1,1.

### Exemple 4 : Evaluation catalytique des silice-alumine SA1, SA2 et SA3 dans un procédé d'oligomérisation avec conversion poussée (deuxième mode de réalisation).

Une coupe C4 oléfinique issue d'une unité de vapocraquage est soumise à un traitement d'hydrogénation sélective dans le but d'éliminer le butadiène, puis séchée sur un tamis moléculaire de type 13X pour éliminer les traces de soufre et d'eau.

La composition de la charge à l'issue de ces traitements est la suivante :

| Composition de la charge (% poids) | |
|---|---|
| Isobutane | 1,55 |
| n-butane | 7,74 |
| isobutène | 39,89 |
| 1-butène | 28,64 |
| Σ 2-butènes | 22,18 |

Cette charge est envoyée dans un réacteur isotherme d'oligomérisation contenant le catalyseur à base de silice-alumine SA1, SA2 ou SA3. Les conditions opératoires sont les suivantes :

| Catalyseur | SA1 Conforme | SA2 Non-conforme | SA3 Non -conforme |
|---|---|---|---|
| Pression | 6,0 MPa | 6,0 MPa | 6,0 MPa |
| Température | 130°C | 130°C | 130°C |
| VVH | 2 h⁻¹ | 2 h⁻¹ | 2 h⁻¹ |

En sortie de réacteur d'oligomérisation la composition massique de l'effluent est la suivante :

| Catalyseur | SA1 Conforme | SA2 Non-conforme | SA3 Non -conforme |
|---|---|---|---|
| Composition de l'effluent (% poids) | | | |
| Isobutane | 3,58 | 3,64 | 3,43 |
| n-butane | 8,01 | 7,89 | 7,82 |
| isobutène | - | - | - |
| 1-butène | 0,23 | 0,26 | 0,26 |
| Σ 2-butènes | 5,77 | 6,81 | 6,80 |
| polymère C5+ | 82,41 | 81,40 | 81,69 |

L'utilisation du catalyseur SA1 conduit à un rendement en polymère C5+ plus important qu'avec les catalyseurs SA2 et SA3. Ladite fraction C5+ est utilisable comme base essence. Le catalyseur SA1 est donc plus sélectif que les catalyseurs SA2 et SA3.

### Exemple 5 : Évaluation catalytique des silice-alumines SA1, SA2 et SA3 dans un procédé d'oligomérisation avec conversion modérée (premier mode de réalisation)

Une coupe C4 oléfinique issue d'une unité de vapocraquage est soumise à un traitement d'hydrogénation sélective destiné à en éliminer le butadiène, puis séchée sur un tamis moléculaire de type 13X pour éliminer les traces de soufre et d'eau. La composition de la charge à l'issue de ces traitements est la suivante :

| Composition de la charge (% poids) | |
|---|---|
| Isobutane | 1,50 |
| n-butane | 6,63 |
| isobutène | 49,48 |
| 1-butène | 27,86 |
| Σ 2-butènes | 14,53 |

Cette charge est envoyée dans un réacteur isotherme d'oligomérisation contenant le catalyseur à base de silice-alumine SA1, SA2 ou SA3. Les conditions opératoires sont les suivantes :

| Catalyseur | SA1 | SA2 | SA3 |
|---|---|---|---|
| Pression | 2,0 MPa | 2,0 MPa | 2,0 MPa |
| Température | 80°C | 80°C | 80°C |
| VVH | 0,5 h⁻¹ | 0,5 h⁻¹ | 0,5 h⁻¹ |

En sortie de réacteur d'oligomérisation la composition massique de l'effluent est la suivante :

| Catalyseur | SA1 | SA2 | SA3 |
|---|---|---|---|
| Composition de l'effluent (% poids) | | | |
| Isobutane | 1,58 | 1,57 | 1,58 |
| n-butane | 6,69 | 6,64 | 6,68 |
| isobutène | 0,37 | 0,41 | 0,40 |
| 1-butène | 20,10 | 22,90 | 22,61 |
| Σ 2-butènes | 21,36 | 19,01 | 19,24 |
| polymère C5+ | 49,90 | 49,47 | 49,49 |

L'utilisation du catalyseur SA1 conduit à un rendement en polymère C5+ plus important qu'avec les catalyseurs SA2 et SA3. Ladite fraction C5+ est utilisable comme base essence. Le catalyseur SA1 est donc plus sélectif que les catalyseurs SA2 et SA3.

### Exemple 6 : Evaluation catalytique des catalyseurs à base de silice-alumine SA1, SA2 et SA3 dans un procédé d'oligomérisation avec conversion poussée (deuxième mode de réalisation)

Une coupe C4 oléfinique issue d'une unité de craquage catalytique est soumise à un traitement d'hydrogénation sélective dans le but d'éliminer le butadiène, puis séchée sur un tamis moléculaire de type 13X pour éliminer les traces de soufre et d'eau.

La composition de la charge à l'issue de ces traitements est la suivante :

| Composition de la charge (% poids) | |
|---|---|
| Isobutane | 29,10 |
| n-butane | 11,45 |
| isobutène | 14,22 |
| 1-butène | 14,20 |
| Σ 2-butènes | 31,03 |

Cette charge est envoyée dans un réacteur isotherme d'oligomérisation contenant le catalyseur à base de silice-alumine SA1, SA2 ou SA3. Les conditions opératoires sont les suivantes :

| Catalyseur | SA1 | SA2 | SA3 |
|---|---|---|---|
| Pression | 6,0 MPa | 6,0 MPa | 6,0 MPa |
| Température | 125°C | 125°C | 125°C |
| VVH | 2 h⁻¹ | 2 h⁻¹ | 2 h⁻¹ |

En sortie de réacteur d'oligomérisation la composition massique de l'effluent est la suivante :

| Catalyseur | SA1 | SA2 | SA3 |
|---|---|---|---|
| Composition de l'effluent (% poids) | | | |
| Isobutane | 29.38 | 29,42 | 29,30 |
| n-butane | 11.43 | 11,45 | 11,45 |
| isobutène | 0.08 | 0,08 | 0,31 |
| 1-butène | 1.92 | 2,41 | 4,03 |
| Σ 2-butènes | 22.34 | 23,15 | 36,25 |
| polymère C5+ | 34.85 | 33,49 | 18,66 |

L'utilisation du catalyseur SA1 conduit à un rendement en polymère C5+ plus important qu'avec les catalyseurs SA2 et SA3. Ladite fraction C5+ est utilisable comme base essence. Le catalyseur SA1 est donc plus sélectif que les catalyseurs SA2 et SA3.

## Revendications

1. Procédé d'oligomérisation d'une charge hydrocarbonée oléfinique consistant en la mise en contact de ladite charge avec un catalyseur comprenant une silice - alumine, la teneur massique en silice dudit catalyseur étant comprise entre 5 et 95% poids, ledit catalyseur étant préparé selon un procédé comprenant au moins :
a) le mélange d'au moins un composé aluminique partiellement soluble en milieu acide avec soit au moins un composé silicique totalement soluble dans le mélange réactionnel soit une combinaison formée d'au moins un composé silicique et d'au moins un composé aluminique, lesdits composés silicique et aluminique étant totalement solubles dans le mélange réactionnel, de manière à former un solide précurseur dudit catalyseur
b) le traitement hydrothermal du solide issu de ladite étape a) par calcination sous air humide pendant une durée comprise entre 4 et 7 heures.

2. Procédé selon la revendication 1 tel que ledit catalyseur est constitué intégralement de ladite silice-alumine.

3. Procédé selon la revendication 1 ou la revendication 2 tel que ledit composé aluminique partiellement soluble en milieu acide est choisi dans le groupe des composés aluminiques de formule générale Al₂O₃, nH₂O (n ≤ 5) et ayant une surface spécifique comprise entre 150 et 600 m²/g.

4. Procédé selon l'une des revendications 1 à 3 tel que pour la mise en oeuvre de ladite étape a), on mélange au moins un composé aluminique partiellement soluble en milieu acide avec au moins un composé silicique totalement soluble dans le mélange réactionnel.

5. Procédé selon la revendication 4 tel que les sources du composé silicique totalement soluble sont choisies dans le groupe formé par l'acide silicique, les solutions colloïdales d'acide silicique, les silicates alcalins hydrosolubles et les sels cationiques de silicium, le Ludox® sous forme ammoniacale ou sous forme alcaline et les silicates d'ammonium quaternaire.

6. Procédé selon l'une des revendications 1 à 3 tel que pour la mise en oeuvre de ladite étape a), on mélange au moins un composé aluminique partiellement soluble en milieu acide avec une combinaison formée d'au moins un composé silicique et d'au moins un composé aluminique, lesdits composés silicique et aluminique étant totalement solubles dans le mélange réactionnel.

7. Procédé selon la revendication 6 tel qu'on utilise des silice-alumines hydratées totalement solubles en tant que source de ladite combinaison.

8. Procédé selon l'une des revendications 1 à 7 tel que ledit traitement hydrothermal par calcination sous air humide est réalisé à une température comprise entre 600 et 1100°C.

9. Procédé selon la revendication 8 tel que ladite calcination sous air humide est réalisée en présence d'une teneur en vapeur d'eau supérieure à 20 g d'eau par kg d'air sec.

10. Procédé selon l'une des revendications 1 à 9 tel que ladite charge hydrocarbonée oléfinique est une coupe C3 oléfinique comprenant au moins 90% poids de propylène et de propane.

11. Procédé selon l'une des revendications 1 à 9 tel que ladite charge hydrocarbonée oléfinique est une coupe C3 - C4 oléfinique.

12. Procédé selon l'une des revendications 1 à 9 tel que ladite charge hydrocarbonée oléfinique est une coupe C4 oléfinique comprenant à plus de 90% poids de l'isobutane, du n-butane, du 1-butène, des 2-butènes et de l'isobutène.

13. Procédé selon l'une des revendications 1 à 9 tel que ladite charge hydrocarbonée oléfinique est une coupe C5 oléfinique.

## Claims

1. A process for oligomerizing an olefinic hydrocarbon feed, consisting of bringing said feed into contact with a catalyst comprising a silica-alumina, the silica content of said catalyst being in the range 5% to 95% by weight, said catalyst being prepared using a process comprising at least:
a) mixing at least one alumina compound which is partially soluble in an acid medium with either at least one silica compound which is completely soluble in the reaction mixture or a combination formed by at least one silica compound and at least one alumina compound, said silica and alumina compounds being completely soluble in the reaction mixture, in order to form a solid precursor of said catalyst;
b) hydrothermal treatment of the solid derived from step a) by calcining in moist air for a period in the range 4 to 7 hours.

2. A process according to claim 1, in which said catalyst is wholly constituted by said silica-alumina.

3. A process according to claim 1 or claim 2, in which said alumina compound which is partially soluble in an acid medium is selected from the group of alumina compounds with general formula Al₂O₃,nH₂O (n ≤ 5) and having a specific surface area in the range 150 to 600 m²/g.

4. A process according to one of claims 1 to 3, in which in order to carry out said step a), at least one alumina compound which is partially soluble in an acid medium is mixed with at least one silica compound which is completely soluble in the reaction mixture.

5. A process according to claim 4, in which the sources of the completely soluble silica compound are selected from the group formed by silicic acid, colloidal silicic acid solutions, hydrosoluble alkaline silicates and cationic silicon salts, Ludox® in its ammoniacal form or in its alkaline form and quaternary ammonium silicates.

6. A process according to one of claims 1 to 3 in which, in order to carry out said step a), at least one alumina compound which is partially soluble in an acid medium is mixed with a combination formed by at least one silica compound and at least one alumina compound, said silica and alumina compounds being completely soluble in the reaction mixture.

7. A process according to claim 6, in which completely soluble hydrated silica-aluminas are used as the source of said combination.

8. A process according to one of claims 1 to 7, in which said hydrothermal treatment by calcining in moist air is carried out at a temperature in the range 600°C to 1100°C.

9. A process according to claim 8, in which said calcining in moist air is carried out in the presence of an amount of steam of more than 20 g of water per kg of dry air.

10. A process according to one of claims 1 to 9, in which said olefinic hydrocarbon feed is an olefinic C3 cut comprising at least 90% by weight of propylene and propane.

11. A process according to one of claims 1 to 9, in which said olefinic hydrocarbon feed is an olefinic C3-C4 cut.

12. A process according to one of claims 1 to 9, in which said olefinic hydrocarbon feed is an olefinic C4 cut comprising more than 90% by weight of isobutane, n-butane, 1-butene, 2-butenes and isobutene.

13. A process according to one of claims 1 to 9, in which said olefinic hydrocarbon feed is an olefinic C5 cut.

## Patentansprüche

1. Verfahren zur Oligomerisierung einer Charge aus olefinischen Kohlenwasserstoffen, das darin besteht, diese Charge mit einem Katalysator in Kontakt zu bringen, der ein Siliziumdioxid - Aluminiumoxid umfasst, wobei der Gewichtsanteil an Siliziumdioxid bei diesem Katalysator im Bereich von 5 bis 95 % liegt, wobei der Katalysator nach einem Verfahren hergestellt wird, das mindestens Folgendes umfasst:
a) Mischen mindestens einer in saurem Milieu teilweise löslichen Aluminiumoxidverbindung, entweder mit mindestens einer Siliziumdioxidverbindung, die vollständig in der Reaktionsmischung löslich ist, oder mit einer Kombination, die aus mindestens einer Siliziumdioxidverbindung und mindestens einer Aluminiumoxidverbindung gebildet ist, wobei die Siliziumdioxid- und Aluminiumoxidverbindungen vollständig in der Reaktionsmischung löslich sind, sodass eine feste Vorläufersubstanz des Katalysators gebildet wird
b) hydrothermale Behandlung des Feststoffs, der aus dem Schritt a) stammt, durch Kalzinieren in feuchter Luft während einer Dauer im Bereich von 4 bis 7 Stunden.

2. Verfahren gemäß dem Anspruch 1, derart, dass der Katalysator vollständig aus dem Siliziumdioxid-Aluminiumoxid besteht.

3. Verfahren nach Anspruch 1 oder Anspruch 2, derart, dass die teilweise in saurem Milieu lösliche Aluminiumoxidverbindung aus der Gruppe der Aluminiumoxidverbindungen der allgemeinen Formel Al₂O₃, nH₂O (n ≤ 5) ausgewählt ist und eine spezifische Oberfläche im Bereich von 150 bis 600 m²/g hat.

4. Verfahren nach einem der Ansprüche 1 bis 3, derart, dass zur Durchführung des Schrittes a) mindestens eine teilweise in saurem Milieu lösliche Aluminiumoxidverbindung mit mindestens einer vollständig in der Reaktionsmischung löslichen Siliziumdioxidverbindung gemischt wird.

5. Verfahren nach Anspruch 4, derart, dass die Quellen der vollständig löslichen Siliziumdioxidverbindung aus der Gruppe ausgewählt sind, die aus Kieselsäure, kolloidalen Kieselsäurelösungen, wasserlöslichen alkalischen Silikaten und kationischen Siliciumsalzen, Ludox® in ammoniakalischer Form oder alkalischer Form und quartären Ammoniumsalzen gebildet ist.

6. Verfahren nach einem der Ansprüche 1 bis 3, derart, dass zur Durchführung des Schrittes a) mindestens eine teilweise in saurem Milieu lösliche Aluminiumoxidverbindung mit einer Kombination, die aus mindestens einer Siliziumdioxidverbindung und mindestens einer Aluminiumoxidverbindung gemischt wird, wobei die Siliziumdioxid- und Aluminiumoxidverbindungen vollständig in der Reaktionsmischung löslich sind.

7. Verfahren nach Anspruch 6, derart, dass vollständig lösliche hydratierte Siliziumdioxide-Aluminiumoxide als Quelle der Kombination verwendet werden.

8. Verfahren nach einem der Ansprüche 1 bis 7, derart, dass die hydrothermale Behandlung durch Kalzinieren in feuchter Luft bei einer Temperatur im Bereich von 600 bis 1100 °C durchgeführt wird.

9. Verfahren nach Anspruch 8, derart, dass das Kalzinieren in feuchter Luft in Gegenwart eines Gehaltes an Wasserdampf von mehr als 20 g Wasser pro kg trockener Luft durchgeführt wird.

10. Verfahren nach einem der Ansprüche 1 bis 9, derart, dass es sich bei der Charge olefinischer Kohlenwasserstoffe um eine olefinische C3 Fraktion handelt, die mindestens 90 Gew.-% an Propylen und an Propan umfasst.

11. Verfahren nach einem der Ansprüche 1 bis 9, derart, dass es sich bei der Charge olefinischer Kohlenwasserstoffe um eine olefinische C3-C4 Fraktion handelt.

12. Verfahren nach einem der Ansprüche 1 bis 9, derart, dass es sich bei der Charge olefinischer Kohlenwasserstoffe um eine olefinische C4 Fraktion handelt, die zu mehr als 90 Gew.-% Isobutan, n-Butan, 1-Buten, 2-Butene und Isobuten umfasst.

13. Verfahren nach einem der Ansprüche 1 bis 9, derart, dass es sich bei der Charge olefinischer Kohlenwasserstoffe um eine olefinische C5 Fraktion handelt.
